# EUROPÄISCHE PATENTSCHRIFT

(11) **EP 3 562 378 B1**
(45) Veröffentlichungstag und Bekanntmachung des Hinweises auf die Patenterteilung: **21.08.2024**
(21) Anmeldenummer: 17844618.3
(22) Anmeldetag: 29.12.2017
(51) Int. Cl.: A61B 3/16, A61B 3/12, A61B 3/14

(54) **VERFAHREN ZUR BESTIMMUNG DES AUFTRETENS EINES GEFÄSSKOLLAPSES BEI EINEM BLUTGEFÄSS IM ODER AM AUGE SOWIE HALTEEINRICHTUNG UND OPHTHALMODYNAMOMETRIEANORDNUNG**
METHOD FOR DETERMINING THE OCCURRENCE OF A VASCULAR COLLAPSE IN A BLOOD VESSEL IN OR ON THE EYE, AND HOLDING DEVICE AND OPHTHALMIC DYNAMOMETRIC ARRANGEMENT
PROCÉDÉ PERMETTANT DE DÉTERMINER L'APPARITION D'UN COLLAPSUS VASCULAIRE AU NIVEAU D'UN VAISSEAU SANGUIN DANS OU SUR L'OEIL, AINSI QUE DISPOSITIF DE RETENUE ET SYSTÈME D'OPHTALMO-DYNAMOMÉTRIE

(30) Priorität: 30.12.2016 DE 102016015577; 22.02.2017 DE 102017001677; 06.10.2017 DE 102017123241
(43) Veröffentlichungstag der Anmeldung: 06.11.2019
(73) Patentinhaber: I-Dynamometer GmbH, 46397 Bocholt (DE)
(72) Erfinder: MEYER-SCHWICKERATH, Rolf, 46397 Bocholt (DE)
(74) Vertreter: Schleitzer, Dirk-Karsten
(86) Internationale Anmeldenummer: PCT/EP2017/025374
(87) Internationale Veröffentlichungsnummer: WO 2018/121886

(56) Entgegenhaltungen:
- US-A1- 2002 049 389
- US-A1- 2004 186 367
- US-A1- 2004 230 124
- US-A1- 2010 152 565
- US-A1- 2010 331 684
- US-A1- 2013 144 185

## Beschreibung

Die Erfindung ist auf eine Halteeinrichtung zur Verwendung bei der Ophthalmodynamometrie und/oder bei einem Verfahren der vorgenannten Art sowie auf eine Ophthalmodynamometrieanordnung zur Bestimmung des Auftretens eines Gefäßkollapses bei einem Blutgefäß im oder am Auge gerichtet.

Zur Bestimmung des Gefäßdrucks in Blutgefäßen im oder am Auge wird bei einer üblichen Methode ein Kontaktglas vom Augenarzt auf das Auge aufgesetzt und die Blutgefäße im Augenhintergrund durch das Kontaktglas beobachtet. Mittels des Kontaktglases wird nun zunehmend ein Druck auf das Auge ausgeübt bis ein erster Gefäßkollaps bei dem beobachteten arteriellen Gefäß auftritt. Aus dem ausgeübten Druck in diesem Moment kann der Arzt einen diastolischen arteriellen Blutdruckwert ermitteln. Wird der Druck auf das Auge weiter erhöht, tritt bei einem bestimmten Druck ein vollständiger Gefäßkollaps auf. Dieser Moment markiert das Erreichen des systolischen arteriellen Blutdrucks im betrachteten Gefäß. In gleicher Weise wird verfahren, um den Gefäß-Innendruck der Venen zu bestimmen. Hier gibt es aber keinen diastolischen und systolischen Druck, da es sich auf der Venenseite um ein Niederdruck-System handelt. Bei den Venen wird der Druck ermittelt, bei dem der Venenkollaps beginnt. Es kann auch die Feststellung getroffen werden, das bei vorhandenem Augeninnendruck bereits ein spontaner Venenkollaps vorliegt. Aus dem Venendruck kann der Hirndruck bestimmt werden.

Die Kontakt-Glas Methode zur Druckbestimmung, bzw. um das Auftreten eines Gefäßkollapses im oder am Auge zu ermitteln, geht mit einer Reihe von Nachteilen einher. Zunächst ist das Aufsetzen eines Kontaktglases für die zu untersuchende Person oder das zu untersuchende Tier oftmals unangenehm und kann zu Schmerzen oder Irritationen im Bereich des Auges führen. Ferner liegt eine Fehlerquelle in der von Arzt zu Arzt unterschiedlichen Art und Weise, wie das Kontaktglas aufgesetzt und geführt wird und im unterschiedlichen Vermögen des Arztes, das Auftreten eines Gefäßkollapses im Bereich des Augenhintergrunds sicher zu erkennen. Es besteht zudem das Problem, dass durch die Druckausübung mittels des Kontaktglases der Augapfel in die Augenhöhle, die sogenannte Orbita, hineingedrückt wird, wodurch der orbitale Venendruck bereits durch die Messung wesentlich erhöht und die Messung als solche somit verfälscht werden kann.

Zur Erhöhung der Reproduzierbarkeit kann die Messung gegebenenfalls zumindest teilweise automatisiert werden. In diesem Fall erfolgt die Bestimmung des Auftretens des Gefäßkollapses nicht durch die Betrachtung durch den Arzt, sondern durch Abbildung des Augenhintergrunds bzw. der dort befindlichen Gefäße mittels einer Kamera.

Ebenso, wie auch bei der unmittelbaren Betrachtung des Augenhintergrunds durch den Arzt, ist hierbei jedoch problematisch, dass aufgrund der Lage der betreffenden Gefäße der Betrachtungswinkel relativ ungünstig ist, da teilweise nahezu axial auf das Gefäß im Bereich der Eintrittsstelle des Sehnervs in den Augapfel, der sogenannten Papille, geblickt wird. Bereits für einen erfahrenen Augenarzt gestaltet sich die sichere Bestimmung des Gefäßkollapses in diesem Bereich daher unter Umständen schwierig. Für eine automatisierte Erkennung eines Gefäßkollapses anhand von aufgenommenen Bilddaten stellt die Lage der Gefäße am Augapfel eine entsprechend größere Hürde dar.

Eine Vorrichtung und ein Verfahren zur Untersuchung physikalischer und chemischer Parameter an einem Auge sind beispielsweise auch aus US 2002/0049389 A1 bekannt. Dort wird unter anderem auf die Verwendung eines Kontaktelements und auf tonometrische Untersuchungen eingegangen.

Aus US 2010/0331684 A1 ist darüber hinaus eine Vorrichtung und ein Verfahren zur nicht-invasiven Bestimmung des Hirndrucks mittels Ultraschall-Doppler-Messungen am Auge bekannt. Hierbei wird Druck auf das Auge mittels eines aufblasbaren Kissens ausgeübt.

Das Dokument US 2010/0152565 A1 offenbart ferner eine Vorrichtung zum Einsatz bei Untersuchen des Augeninnendrucks. Es wird dort eine Halteeinrichtung beschrieben, die es erlaubt, einen Messfühler zur Druckausübung auf das Auge einer Person exakt vor dem Auge positionieren und zu führen.

Vor diesem Hintergrund ist es Aufgabe der vorliegenden Erfindung, eine Möglichkeit zur verlässlicheren und insbesondere automatisierbaren Bestimmung des Auftretens eines Gefäßkollapses bei einem Auge bereitzustellen.

Die vorgenannte Aufgabe wird erfindungsgemäß durch eine Halteeinrichtung gemäß Anspruch 1 gelöst. Vorteilhafte Weiterbildungen sind jeweils Gegenstand der Unteransprüche.

Bei einem Verfahren wird der Gefäßkollaps im Auge nicht ausschließlich visuell, das heißt durch äußere Beobachtung des Gefäßes durch den Arzt oder mittels einer Kamera oder einer ähnlichen Abbildungseinrichtung, bestimmt, sondern aus einer gemessenen zeitlichen Änderung des Blutflusses, insbesondere der mittleren Strömungsgeschwindigkeit des Blutes, im betreffenden Blutgefäß. Bei entsprechenden Versuchen wurde überraschenderweise durch den Erfinder festgestellt, dass beim Auftreten eines Gefäßkollapses der Blutfluss in dem betreffenden Gefäß in charakteristischer Weise sistiert. Durch eine zeitlich aufgelöste Messung des Blutflusses und einer hierdurch ermöglichten Bestimmung einer zeitlichen Änderung des Blutflusses in Form eines temporären Sistierens lässt sich daher auf verlässliche Weise das Auftreten eines Gefäßkollapses bei einem Gefäß des Auges detektieren.

Die auf der zeitlichen Blutflussänderung basierende Bestimmung des Auftretens eines Gefäßkollapses besitzt gegenüber visuellen Methoden den Vorteil, dass selbst bei axialer eingeschränkter Sicht auf das kollabierende Gefäß ein verlässlicher Schluss auf das Vorliegen eines Gefäßkollapses möglich ist. Abhängig vom eingesetzten Verfahren ist es zur Bestimmung des Blutflusses bzw. dessen zeitlicher Änderung sogar von Vorteil, die Messung nicht in axialer Richtung des Gefä-ßes, sondern am Sehnervenrand, wo die Gefäße horizontal zum diagnostischen Strahl verlaufen, vorzunehmen. Die Messung kann in oder entgegen der Flussrichtung des Blutes im Inneren des Gefäßes vorgenommen werden. Letztlich ist dies insbesondere bei Messmethoden von Vorteil, die auf einem Phasenschub eines rückgestreuten gegenüber einem einfallenden Signal basieren, worauf im Folgenden weiter eingegangen werden wird.

Bevorzugt wird der Blutfluss mittels eines optischen Verfahrens gemessen, wobei die Methode der optischen Kohärenztomographie (OCT) besonders bevorzugt ist. Zur Geschwindigkeitsbestimmung bei untersuchten Objekten ist dabei insbesondere die sogenannte Doppler-OCT, d.h. die optische Kohärenztomographie in Duplex-Technik, eine bevorzugte Methode.

Bei der Methode der OCT wird nach dem Grundprinzip eines Interferometers ein Messlichtstrahl mit einem Referenzlichtstrahl in Beziehung gesetzt, so dass sich aus der Interferenz der beiden Strahlen Informationen über die mit dem Messlichtstrahl untersuchten Strukturen erhalten lassen. Bei der OCT werden üblicherweise Lichtquellen mit vergleichsweise kleiner Kohärenzlänge eingesetzt. Bei geeigneter Wahl des Spektrums der Lichtquelle vermag die Lichtstrahlung bis zu einer gewissen Tiefe in Gewebsschichten einzudringen und auf diese Weise eine Tiefeninformation des untersuchten Gewebes zu liefern. Dabei besitzt die OCT den Vorteil, dass die laterale und die longitudinale Auflösung voneinander entkoppelt sind, da die laterale Auflösung von der numerischen Apertur einer Abbildungsoptik abhängt, während die longitudinale Auflösung primär vom Spektrum bzw. der spektralen Breite des Lichts der Lichtquelle abhängig ist.

Mittels der Methode der OCT lassen sich demnach tomographisch detaillierte dreidimensionale Abbildungen von Gewebsstrukturen erzeugen. Auf diesem Grund wird die Methode der OCT am Auge bereits seit einiger Zeit zur Angiographie, das heißt zur Darstellung vorhandener Gefäßstrukturen genutzt die sogenannte Angio-OCT.

Wie nun überraschend festgestellt wurde, kann mittels einer OCT-Messung zum einen auch der Blutfluss im Inneren der Gefäße dargestellt werden und zum anderen können mittels zeitlich aufgelöster OCT auch Veränderungen dieses Blutflusses mit hoher Genauigkeit detektiert werden. Ein Sistieren des Blutflusses, wie es beim Vorliegen eines Gefäßkollapses auftritt, kann somit durch die Methode der OCT mit hoher Präzision detektiert werden. Somit ist das Auftreten eines Gefäßkollapses beim Auge auf verlässliche und nicht-invasive Weise bestimmbar.

Es versteht sich, dass alternativ oder zusätzlich zur grundsätzlich bevorzugten Methode der optischen Kohärenztomographie auch weitere Verfahren zur Flussmessung geeignet und erfindungsgemäß einsetzbar sind. Hierzu gehören insbesondere die Methode der Scanning Laser Doppler Flowmetrie (SLDF) und die Laser Speckle Flowmetrie.

Bei einem Verfahren zur Bestimmung des Auftretens eines Gefäßkollapses bei einem arteriellen Gefäß im oder am Auge wird Druck auf das Auge bis zum Erreichen des Gefäßkollapses ausgeübt und der Gefäßkollaps aus einer gemessenen zeitlichen Änderung des Blutflusses im Blutgefäß bestimmt. Im Gegensatz zur Zentral-Arterie handelt es sich im Venensystem um ein Niederdrucksystem. In den meisten Fällen ist ein ähnliches Kollapsverhalten der Zentralvene wie bei der Zentral-Arterie zu erwarten. Hier gelten die gleichen Bedingungen für den Gefäßkollaps wie bei der Zentral-Arterie. Auch hier wird der Gefäßkollaps aus einer gemessenen zeitlichen Änderung des Blutflusses im Blutgefäß bestimmt. Da der Venendruck vom Hirndruck abhängig ist, ist mit Hilfe des Venendrucks auch eine Hirndruckmessung möglich. Das Verfahren lässt die Bestimmung eines spontanen Venenkollapses zur Feststellung eines nicht-erhöhten Hirndruckes zu, auch ohne dynamometrisches Verfahren. Lediglich in den Fällen, in denen kein spontaner Venenkollaps vorliegt, ist es erforderlich, Druck auf das Auge bis zum Erreichen des Venenkollapses auszuüben. Im Ergebnis kann vorgesehen sein, zum Erreichen eines arteriellen Gefäßkollapses oder eines nicht-spontanen Venenkollapses Druck auf das Auge auszuüben, bis der Gefäßkollaps eintritt. In den Fällen, in denen ein spontaner Venenkollaps vorliegt, ist die Druckausübung auf das Auge dagegen nicht erforderlich. Auch die Feststellung eines spontanen Gefäßkollapses im Venensystem gilt als dynamometrische Feststellung, weil hier der spontane Augeninnendruck ausreichend ist, um einen spontanen Venenkollaps zu erzeugen.

Kompressionsort radiär zum Augapfel-Äquator: Die Druckausübung auf den Augapfel, um letztlich den Gefäßkollaps bei dem beobachteten arteriellen Gefäß hervorzurufen, erfolgt seitlich bzw. aus seitlicher Richtung, das heißt zumindest im wesentlichen quer zur Blickachse eines zu untersuchenden Auges. Zum einen ist somit der Beobachtungsweg für eine optische Blutflussbestimmung frei. Zum anderen wird durch den seitlichen Druck auf das Auge eine Verfälschung infolge eines Hineindrückens des Augapfels in die Augenhöhle und der damit verbundenen Kompression der Augenhöhle bzw. einer Erhöhung des Gewebedrucks in der Augenhöhle erheblich reduziert.

Kompressions-Stelle Lider: Die Druckausübung auf das Auge erfolgt mittelbar, das heißt nicht direkt auf den Augapfel. Wird der Druck über das obere und/oder das untere Augenlid des Auges auf den Augapfel ausgeübt, wird der systematische Fehler aufgrund des ausgeübten Drucks bei einer Gefäßdruckbestimmung weiter minimiert, da das Lid letztlich im niedrigen Druckbereich eine puffernde Wirkung besitzt, was gerade bei der Ankopplung des Druck-Kopfes des Dynamometers einen erheblichen Vorteil darstellt. Im Gegensatz dazu stellt eine direkte Ankopplung am Augapfel eine unkontrollierte primäre Druckerhöhung dar, ein Problem das allen anderen Methoden, die direkt Druck auf den Augapfel ausüben - auch der Kontakt-Glas-Dynamometrie - anhaftet. Eine besonders geeignete Stelle, um den erforderlichen Druck zu applizieren, liegt dabei im Bereich des temporalen Lidwinkels, weil hier die Druck-Applikation radiär zum Augapfel-Äquator erfolgt.

Der auf das Auge ausgeübte Druck kann manuell und/oder automatisch vorgegeben werden, wobei eine automatische Einstellung des Drucks insbesondere von einer Mess- und Auswertungseinrichtung vorgenommen wird. Der tatsächlich auf das Auge ausgeübte Druck kann ferner durch einen Sensor gemessen oder anderweitig insbesondere von der Mess- und Auswertungseinrichtung ermittelt werden. Alternativ oder zusätzlich kann zudem eine Visualisierung des ausgeübten Drucks erfolgen. Dies ist beispielsweise über eine mechanische Skala oder eine digitale Anzeige möglich.

Insbesondere zur Steuerung und/oder zum Auslesen von Messdaten, wie beispielsweise einem auf das Auge ausgeübten Druck, kann eine Datenübertragung zwischen dem Druckgeber, einem Sensor und/oder der Mess- und Auswertungseinrichtung bzw. einer Messanordnung im Allgemeinen erfolgen. Die übertragenen Daten umfassen dementsprechend vorzugsweise Steuerdaten und/oder Messdaten, insbesondere einen Druckwert. Bei der Messanordnung handelt es sich vorzugsweise um eine optische Kohärenztomographie-Anordnung und/oder eine Scanning Laser Doppler Flowmetrie-Anordnung und/oder eine Laser Speckle Flowmetrie-Anordnung. Durch eine direkte Datenübertragung zwischen einer Einrichtung zur Steuerung und/oder Auswertung und dem Druckgeber bzw. Sensor wird letztlich erst eine Automatisierung des Verfahrens ermöglicht. Die Datenübertragung erfolgt dabei bevorzugt in bidirektionaler Weise.

Die Datenübertragung zwischen dem Druckgeber, dem Sensor und/oder der Mess- und Auswertungseinrichtung bzw. der Messanordnung läuft vorzugsweise drahtlos ab. Hierdurch wird der Aufwand in Bezug auf den Anschluss und die Inbetriebnahme einer für das erfindungsgemäße Verfahren ausgebildeten Vorrichtung erheblich reduziert. Eine drahtlose Verbindung ermöglicht ferner eine Kontrolle und Steuerung des Verfahrens, ohne eine zu untersuchende Person in ihrer Bewegungsfreiheit übermäßig einzuschränken. Die Datenübertragung erfolgt hierbei insbesondere über verbreitete Protokolle, wie beispielsweise Bluetooth und/oder Wireless LAN. Dies gewährleistet neben geringen Kosten für entsprechende Module eine hohe Kompatibilität verschiedener Hardware-Komponenten, die im Rahmen des erfindungsgemäßen Verfahrens miteinander zusammenwirken.

In einer bevorzugten Ausgestaltung des Verfahrens ist ferner eine Fernsteuerung bzw. -auslesung des Druckgebers, des Sensors und/oder der Mess- und Auswertungseinrichtung, insbesondere über eine Datenfernübertragungsleitung, beispielsweise über das Internet, vorgesehen. Dies erlaubt zum einen medizinischem Fachpersonal die Ausführung bzw. Steuerung des Verfahrens, ohne am Anwendungsort persönlich anwesend zu sein. Zum anderen kann mittels einer Datenfernübertragung eine technische Diagnose und/oder eine Einstellung von Parametern, beispielsweise die Einspielung von Updates, durch technisches Service-Personal aus der Ferne erfolgen.

Aus einem nach dem Verfahren verlässlich bestimmten Auftreten eines Gefäßkollapses bei einem Gefäß des Auges kann bei einer bevorzugten Ausgestaltung des Verfahrens in der Folge der Gefäßdruck des betrachteten Gefäßes ermittelt werden. Hierzu wird der gemessene auf das Auge ausgeübte Druck im Moment des Auftretens des Gefäßkollapses zugrunde gelegt. Auf ähnliche Weise lässt sich ggf. durch eine entsprechende Kalibration aus dem ausgeübten Druck beim Auftreten des Gefäßkollapses über den Gefäßdruck auch auf den Hirndruck schließen. Hierzu ist insbesondere die Bestimmung des venösen Blutdrucks am Auge von Bedeutung. Eine sichere Bestimmung eines venösen Gefäßkollapses am Auge ist mit bekannten visuellen Methoden allerdings besonders problematisch. Zudem ist der venöse Gefäßdruck insbesondere für störende Einflüsse auf die Messung infolge einer durch die Messung verursachten Gewebsdruckveränderung anfällig. Hier setzt letztlich das Verfahren an, indem die Vorteile aus einer auf den Blutfluss bzw. der Blutflussänderung basierenden Bestimmung des Gefäßkollapses zum Tragen kommen.

Die Erfassung des auf das Auge ausgeübten Drucks erfolgt vorzugsweise entkoppelt von der Erzeugung des Drucks, beispielsweise mittels eines Druckgebers. Dies erlaubt eine Messung auch während einer Änderung, insbesondere Steigerung, des Drucks. Andernfalls würde der erfasste Druck durch die kontinuierliche oder schrittweise Druckänderung im System jeweils beeinflusst, was ggf. zu unzuverlässigen oder unbrauchbaren Messdaten führen würde.

Im Fall der Messung des venösen Blutdrucks tritt der Gefäßkollaps beim monophasischen Venenkollaps nur einmalig auf. Ein solches einmaliges Ereignis ist in der Regel nur schwer eindeutig und somit unzuverlässig detektierbar. Diesbezüglich führt eine undulierende Druckausübung zu einem wiederholten Auftreten des Gefäßkollapses. Die Detektierbarkeit des Gefäßkollapses wird auf diese Weise deutlich erhöht. Zu diesem Zweck kann eine entsprechend modulierte Ansteuerung eines Druckgebers vorgesehen sein, so dass eine undulierende Druckausübung automatisiert erfolgen kann. Hierbei kann ein Soll-Mittelwert vorgegeben werden, um den herum der ausgeübte Druck mit einer vorzugsweise einstellbaren Amplitude schwankt. Der Druckbereich, in welchem eine Schwankung erfolgt, liegt vorzugsweise zwischen dem einen Kollaps auslösenden Druckniveau und 3 mmHg, bevorzugt 5 mmHg, niedriger.

Ein Aspekt der Erfindung zur Lösung der vorbezeichneten Aufgabe liegt in einer Halteeinrichtung, insbesondere ausgebildet zur Verwendung bei der Ophthalmodynamometrie bzw. bei einem Verfahren der zuvor beschriebenen Art. Die erfindungsgemäße Halteeinrichtung weist einen Druckgeber oder Dynamometer zum Ausüben eines äußeren Drucks auf ein Auge sowie ein Halteelement zum Halten und/oder Positionieren des Druckgebers relativ zum Auge auf. Die erfindungsgemäße Halteeinrichtung erfüllt zusammen mit dem Druckgeber insbesondere die Funktion einer Dynamometrieanordnung.

Die erfindungsgemäße Halteeinreichtung lässt sich auch zur Bestimmung des Abflusswiderstandes des Augenwassers bei der Okulo-Pressions-Tonometrie (OPT) vorteilhaft einsetzen.

Die erfindungsgemäße Halteeinrichtung bzw. Dynamometrieanordnung erlaubt im Rahmen der Glaukomdiagnostik insbesondere eine Untersuchung des Auges in Bezug auf die Kammerwasserdynamik. Es lässt sich hierbei der Abflusswiderstand des Kammerwassers durch eine Veränderung des Innendrucks des Auges bestimmen. Hierzu kann die Dynamometrieanordnung beispielsweise mit einem Luft-Tonometer und einer Mess- und Auswertungseinrichtung gekoppelt werden.

Zur Bestimmung des Abflusswiderstands kann nun, beispielsweise mittels des Luft-Tonometers, zunächst der Augeninnendruck bestimmt werden. Daraufhin erfolgt eine Druckbeaufschlagung des Auges mittels des Druckgebers des Dynamometers. Nach Aufhebung der Druckbeaufschlagung des Auges erfolgt dann eine weitere Messung des Augeninnendrucks. Der im Nachhinein gemessene Wert wird in der Regel niedriger ausfallen, da Kammerwasser infolge des äußeren Drucks abgeflossen ist. Aus dem Unterschied der beiden ermittelten Druckwerte kann letztlich auf den Abflusswiderstand des Auges für das Kammerwasser geschlossen werden. Je niedriger der Augeninnendruck nach der Druckbelastung ausfällt, desto niedriger ist der Abflusswiderstand, d.h. desto mehr Kammerwasser ist abgeflossen.

Anders als bei der klassischen Dynamographie erfolgt also eine Bestimmung des Augeninnendrucks vor und nach der dynamometrischen Druckbelastung und nicht eine Bestimmung der Abnahme des Augeninnendrucks im Moment der dynamometrischen Druckbelastung. Da unter der Augeninnendruckerhöhung mehr Augenwasser abgeflossen ist, fällt der Augeninnendruck nach der Druckbelastung entsprechend stark ab. Eine Differenzierung zwischen gestörtem und normalem Abfluss in Bezug auf die Abflussleichtigkeit (Fazilität) wird hierdurch erleichtert.

Wenngleich die vorstehend beschriebene Methode der Okulopressions-Tonometrie (OPT) grundsätzlich seit längerem bekannt ist, hat der hohe methodische Aufwand eine weitere Verbreitung bisher unmöglich gemacht. Die vorliegende Erfindung bietet hier eine erhebliche methodische Erleichterung und sogar die Möglichkeit, den Ablauf der OPT-Messung weitgehend zu automatisieren. Auf diese Weise lassen sich zukünftig mit geringem Aufwand und bei vergleichsweise kurzen Untersuchungszeiten wichtige Aussagen über den Abflusswiderstand treffen. Die hat beispielsweise Vorteile in Bezug auf einen möglichen Einsatz im Rahmen einer Therapie-Überwachung.

Eine Untersuchungsanordnung zur Okulopressions-Tonometrie (OPT) umfasst vorzugsweise ein Dynamometrieanordnung mit der erfindungsgemäßen Halteeinrichtung und ein Messgerät zur Ermittlung des Augeninnendrucks, insbesondere ein Luft-Tonometer, sowie eine Auswertungseinrichtung. Mit einer solchen Anordnung lässt sich mit geringem Aufwand und vorzugsweise automatisiert ein gestörter Abfluss des Augenwassers bzw. Kammerwassers diagnostizieren.

Die Erfindung umfasst ferner ein Verfahren zur OPT unter Verwendung der erfindungsgemäßen Halteeinrichtung sowie die Verwendung der erfindungsgemäßen Halteeinrichtung zur Okulopressions-Tonometrie.

Ferner können Blutdrücke der Netzhaut-Arterien und -Venen sowie der Hirndruck bestimmt werden. Hierzu kann die Dynamometrieanordnung mit einem entsprechenden Messgerät, vorzugsweise einem optischen Kohärenztomographen, gekoppelt werden. Das Messgerät dient in diesem Fall als Kollapsdetektor. Eine nachfolgende Auswertung und/oder Ausgabe der Messdaten mittels einer Auswertungs- bzw. Datenverarbeitungseinrichtung gibt Aufschluss über die herrschenden Gefäßdrücke im und am Auge.

Insbesondere durch die Kopplung eines OCT-Geräts und der Dynamometrieanordnung aus der erfindungsgemäßen Halteeinrichtung und einem Druckgeber lässt sich zudem anhand der papillären Mikrozirkulation bestimmen, in welchem Umfang die Sehnerven eine dynamometrische Belastung tolerieren. Bei einem Glaukom wird ein Zusammenhang des Auftretens einer Schädigung der Sehnerven mit einer Störung der papillären Mikrozirkulation vermutet. Die Erfindung kann in diesem Zusammenhang dazu beitragen, wertvolle Hinweise auf die auftretenden, möglicherweise schädigenden Effekte zu liefern.

Als viertes Einsatzfeld stellt sich die Elektro-Physiologische Diagnostik des Auges dar. Eine Kopplung der Dynamometrie unter Verwendung der erfindungsgemäßen Halteeinrichtung mit der Messung visuell evozierter Potentiale (VEP) und/oder der Methode der Elektroretinographie (ERG) ermöglicht eine elektro-physiologische Netzhaut- bzw. Sehnervdiagnostik in Abhängigkeit vom applizierten Druck. Dies kann Aufschluss über die Empfindlichkeit des Sehnerven auf geänderte Druckbedinungen liefern und/oder zur Bestimmung der Netzhautaktivität und ihrer Veränderung bei einer Erhöhung des Augeninnendrucks dienen.

Der Druckgeber ist dabei derart mit dem Halteelement verbunden, dass er bei der bestimmungsgemäßen Verwendung der Halteeinrichtung aus seitlicher Richtung auf das Auge Druck ausüben kann. Die Druckausübung erfolgt dabei über das Oberlid und/oder das Unterlid des Auges, und zwar im Bereich des temporalen Lidwinkels. Hierdurch wird zum einen der von der zu untersuchenden Person oft als unangenehm empfundene unmittelbare Kontakt eines Teils des Druckgebers mit dem Augapfel vermieden. Zum anderen wird durch eine solche mittelbare Druckausübung der Effekt einer leichten Drucksteigerung durch Ankopplung des Dynamometer-Kopfes im Inneren des Auges bereits aufgrund der Drucksonden-Positionierung vermieden oder zumindest reduziert, was andernfalls zu einer Fehlmessung führen könnte.

Insbesondere ist die Halteeinrichtung zur selbsttragenden Befestigung am Kopf eines Patienten ausgebildet. Die Halteeinrichtung ist vorzugsweise am Kopf des Patentienten befestigbar und/oder mit dem Kopf des Patienten derart verbindbar, dass die Halteeinrichtung lediglich über den Kopf des Patienten für eine Druckausübung auf das Auge gehalten ist. Insbesondere unterscheidet sich die erfindungsgemäße Halteeinrichtung von stationären Halteeinrichtungen, die gestellartig aufgebaut und fest mit einem Untergrund, wie einem Behandlungstisch oder dergleichen, verbunden sind und im Stand der Technik zur Halterung von insbesondere optischen Einrichtungen bei der Augenuntersuchung zum Einsatz kommen.

Weiter insbesondere ist die Halteeinrichtung derart ausgebildet, dass im Tragezustand, wenn die Halteeinrichtung am Kopf eines Patienten gehalten bzw. befestigt ist, das Halteelement mit dem Druckgeber auf einer Kopfseite, d.h. seitlich zum Kopf zur Ausübung eines Drucks auf das Auge, angeordnet ist, während ein weiteres Halteelement der Halteeinrichtung auf der anderen Kopfseite gegen den Kopf anliegt, so dass ein Widerlager bei der Druckausübung auf das Auge geschaffen wird.

Zur Druckbestimmung weist die Dynamometrieanordnung, insbesondere die Halteeinrichtung und/oder der Druckgeber vorzugsweise eine insbesondere einen Drucksensor aufweisende Druckbestimmungseinrichtung auf. Die Druckbestimmungseinrichtung ist vorzugsweise entkoppelt von der Druckerzeugung am Druckgeber. Die Druckausübung erfolgt in diesem Fall also unabhängig von einer Druckmesseinheit bzw. einem Drucksensor. Dadurch wird eine Beeinflussung der Messung durch die Druckänderung im Druckgeber vermieden. Eine Messung des Drucks ist somit auch während einer kontinuierlichen oder schrittweisen Druckänderung möglich.

Bei einer besonders bevorzugten Ausgestaltung der erfindungsgemäßen Halteeinrichtung ist diese portabel bzw. selbsttragend ausgebildet. Dies bedeutet, dass die Halteeinrichtung bei der Verwendung, also wenn der Druckgeber in der bestimmungsgemäßen Position relativ zum Auge angeordnet ist, insbesondere einem Menschen oder einem Tier aufgesetzt und von diesem getragen werden kann. Eine tragende mechanische Verbindung zwischen der Halteeinrichtung und einer mit dem Patienten nicht verbundenen stationär bzw. ortsfest angeordneten Einrichtung, wie einem Behandlungstisch oder dergleichen, ist dann nicht vorgesehen.

In einer besonders geeigneten Ausführungsform ist die Halteeinrichtung hierzu nach Art eines Brillengestells ausgebildet. Hierdurch kann eine Person die Halteeinrichtung zur Durchführung eines ophthalmodynamometrischen Verfahrens komfortabel aufsetzen, wobei der Druckgeber ohne größeren Justageaufwand bereits in die für eine Messung bzw. die Druckausübung bei einer Messung korrekte Position gebracht wird. Zudem ist wegen der automatisch korrekten Positionierung des Druckgebers relativ zum Auge bei einer Verwendung der Halteeinrichtung die Durchführung der ophthalmodynamometrischen Messung nicht zwingend vom Arzt vorzunehmen, sondern kann vom Arzt an medizinisches Personal delegiert werden. Eine Brille hat einen definierten Bezug zur Anatomie einer Person. Sie berücksichtigt die grundsätzliche Breite des Kopfes sowie die Pupillendistanz, d.h. den Abstand der Augen voneinander. Mit einem Brillengestell als Halteeinrichtung besteht somit ein fester Bezug zur Anatomie des Gesichts, insbesondere des Augapfels.

Das Halteelement der Halteeinrichtung ist dementsprechend vorzugsweise als Brillenbügel eines Brillengestells ausgebildet. Die Halteeinrichtung kann in diesem Fall in der Art einer Brille auf den Kopf aufgesetzt werden, so dass das Halteelement seitlich am Kopf entlang verläuft und der Druckgeber im Bereich des Auges und seitlich zu diesem angeordnet ist. Es versteht sich, dass die Halteeinrichtung für einen sicheren Sitz vorzugsweise symmetrisch ausgebildet ist, also beispielsweise zwei Brillenbügel aufweist. Der zweite Brillenbügel des Brillengestells bildet dabei ein Widerlager auf der anderen Kopfseite des Patienten, um während der Druckausübung auf das Auge eine stabile Position des Druckgebers zum Auge zu gewährleisten. Es ist zudem nicht erforderlich, dass die Halteeinrichtung tatsächliche Sichtfenster oder gar Brillengläser aufweist. Letztlich ist die einen Kopf umgreifende Form eines Brillengestells der wesentliche Aspekt, der eine solche Form für die erfindungsgemäße Halteeinrichtung besonders geeignet sein lässt.

In einer alternativen Ausführungsform kann die erfindungsgemäße Halteeinrichtung in der Art eines Helmes ausgebildet sein und/oder ein Fixierband zur Befestigung am Kopf eines Trägers der Halteeinrichtung aufweisen. Die äußere Form der Halteeinrichtung und die Art der Befestigung und Positionierung des Druckgebers relativ zum Kopf bzw. Auge des Trägers ist entsprechend der konkreten Anwendungssituation wählbar. Hierbei sind insbesondere Anforderungen an die Festigkeit des Sitzes der Halteeinrichtung, den Tragekomfort, die Tragedauer und ggf. die Zugänglichkeit des Auges für weitere Untersuchungsgeräte zugrunde zu legen.

Ist ein geeignetes Tragegestell bereits vorhanden, beispielsweise in Form einer regelmäßig getragenen Brille bei Brillenträgern, kann die Halteeinrichtung, insbesondere das Halteelement, auch zur Verbindung mit einem Brillenbügel ausgebildet sein. Die eigene Brille eines Brillenträgers ist in der Regel bereits optimal an seine Kopfform angepasst, wodurch ein sicherer Sitz und ein hoher Tragekomfort gewährleistet ist. Die erfindungsgemäße Halteeinrichtung bzw. das Halteelement mit dem damit verbundenen Druckgeber kann in diesem Fall beispielsweise im Bereich des Auges seitlich zu diesem am Brillenbügel oder einem anderen geeigneten Teil des Brillengestells befestigt werden. Hierbei ist eine sichere, jedoch vergleichsweise einfach lösbare Verbindung, beispielsweise mittels eines Halteclips oder durch eine Schraubverbindung, bevorzugt.

Der Druckgeber übt bei der Verwendung Druck auf das Auge über ein entsprechendes Kontaktelement zur Anlage gegen das Auge oder gegen Teile des Auges aus. Das Kontaktelement ist dabei zur Anlage gegen das Oberlid und/oder das Unterlid, bevorzugt im temporalen Lidwinkel, ausgebildet. Durch die Lid-Dynamometrie entfällt die Notwendigkeit einer Oberflächen-Anästhesie des Auges. Dies hat zum einen den Vorteil, dass die Prozedur für die untersuchte Person erheblich angenehmer abläuft. Zum anderen kann die Untersuchung bei Ausbleiben einer Anästhesie vom einem Arzt auf Assistenzpersonal delegiert werden.

Geeignet ist eine Ausgestaltung des Kontaktelements als Knauf, als Stößel und/oder als Kontaktplatte. Die konkrete Form des Kontaktelements hängt dabei letztlich von der konkreten Anwendung ab. Das Kontaktelement kann dabei beispielsweise in Abhängigkeit davon, ob auf das Oberlid, das Unterlid oder beide Lider Druck ausgeübt werden soll, unterschiedlich ausgestaltet sein.

Das Kontaktelement des Druckgebers ist mechanisch, hydraulisch und/oder pneumatisch bewegbar. Insbesondere im Fall eines pneumatisch bewegbaren Kontaktelements weist der Druckgeber vorzugsweise eine Kolben-Zylinder-Einheit zur Bewegung des Kontaktelements auf.

Eine verbesserte Anlage des Kontaktelements gegen einen betreffenden Kontaktbereich am Auge wird erreicht, wenn das Kontaktelement gelenkig gelagert ist. Für eine gelenkige Lagerung des Kontaktelements kann dieses insbesondere mit der Kolbenstange eines Pneumatikzylinders bzw. einer Kolben-Zylinder-Einheit der zuvor beschriebenen Art derart gelenkig verbunden sein, dass das Kontaktelement in einer oder mehreren Richtungen schwenkbar ist.

Insbesondere zur gleichzeitigen Druckausübung auf das Ober- und das Unterlid eines Auges kann das Kontaktelement mehrere, vorzugsweise zwei, getrennte Kontaktabschnitte aufweisen, die ihrerseits zur Anlage gegen das Auge oder gegen Teile des Auges, das heißt insbesondere das Oberlid und/oder das Unterlid eines Auges, bestimmt sind. Die Kontaktabschnitte können alternativ oder zusätzlich zu einer gelenkigen Lagerung des Kontaktelements auch individuell gelenkig und/oder schwenkbar gelagert sein, um eine optimale Anlage gegen den entsprechenden Kontaktbereich des Auges zu gewährleisten. Diese Ausbildung des Dynamometers als Doppelkopf-Dynamometer bringt den Vorteil mit sich, dass die Kraftausübung auf den Augapfel zentriert erfolgt. Scherkräfte, die gerade bei ungeübten Anwendern und insbesondere in Verbindung mit herkömmlichen Dynamometern auftreten können, werden hierdurch auf ein Minimum reduziert.

Die laterale Ausdehnung bzw. der Durchmesser eines Kontaktabschnittes des Druckgebers beträgt vorzugsweise zwischen 2 mm und 10 mm, bevorzugt zwischen 4 mm und 8 mm, besonders bevorzugt zwischen 6 mm und 7 mm. Es ist insbesondere nicht erforderlich, dass ein Kontaktabschnitt in beiden lateralen Dimensionen dieselbe Ausdehnung aufweist. Vielmehr kann erfindungsgemäß auch eine längliche, insbesondere eine länglich-ovale, Form des Kontaktabschnitts vorgesehen sein.

Das Kontaktelement und/oder wenigstens ein Kontaktabschnitt weist in einer bevorzugten Ausführungsform eine gekrümmte und/oder gewölbte Oberfläche auf. Die Krümmung bzw. Wölbung ist dabei insbesondere konvex, d.h. in Richtung der Druckausübung durch den Druckgeber mittig vorspringend, ausgebildet. Es ist hierbei nicht erforderlich, dass das Kontaktelement oder Der Krümmungsradius ist insbesondere geringfügig größer als der übliche Krümmungsradius des Augapfels und beträgt vorzugsweise zwischen 5 mm und 15 mm, bevorzugt zwischen 7 mm und 13 mm, besonders bevorzugt zwischen 9 mm und 11 mm.

Alternativ oder zusätzlich kann das Kontaktelement und/oder einer oder mehrere Kontaktabschnitte auch in Bezug auf den Winkel anpassbar. Es versteht sich, dass der Winkel der Kontaktabschnitte, bezogen auf die Bewegungsrichtung und/oder zueinander, nicht zwingend identisch sein müssen. Es kann beispielsweise eine leichte Abwinkelung eines Kontaktabschnitts gegenüber dem anderen vorgesehen sein. Hierdurch lässt sich das Dynamometer individuell in optimaler Weise an die jeweilige Augenkontur anpassen.

Das Kontaktelement ist vorzugsweise so ausgebildet, dass beim Heranfahren an den Augapfel eine gleichmäßige Anlage an beide Lid-Enden erfolgt, so dass keine stärkeren Drücke auf der Oberseite oder Unterseite entstehen. Bevorzugt ist der Dynamometerkopf, d.h. insbesondere das Kontaktelement des Druckgebers, im kontaktfreien Zustand im Wesentlichen senkrecht ausgerichtet.

Aus hygienischer Sicht ist es von Vorteil, wenn das Kontaktelement und/oder die Kontaktabschnitte austauschbar ausgebildet ist bzw. sind. Durch einen einfachen Wechsel eines benutzten Teils gegen ein neues und vor allem steriles Teil entfällt die Notwendigkeit einer aufwendigen Reinigung und Sterilisation der Elemente, die in unmittelbaren Kontakt mit Körperteilen geraten, zwischen Messungen an verschiedenen Personen. Alternativ oder zusätzlich kann ein austauschbarer Bezug für das Kontaktelement und/oder die Kontaktabschnitte vorgesehen sein, der jeweils vor dem Einsatz der Halteeinrichtung neu aufgezogen wird.

Zum Schutz des Auges oder für besondere Anwendungssituationen, beispielsweise bei Messungen in einem bestimmten Druckbereich, kann der vom Druckgeber ausgeübte Druck auf einen bestimmten Maximalwert begrenzt werden. Hierzu ist der Druckgeber vorzugsweise derart ausgebildet, dass bei Erreichen eines festgelegten und/oder festlegbaren Maximaldrucks auf das Auge automatisch keine weitere Druckerhöhung durch den Druckgeber erfolgt. Dies kann grundsätzlich durch eine Begrenzung des Bewegungsweges des vom Druckgeber bewegten Kontaktelements, beispielsweise mittels eines mechanischen Anschlags, erfolgen. Erfindungsgemäß möglich sind darüber hinaus auch Lösungen, die auf einer Messung des ausgeübten Drucks und einer automatischen Abschaltung und/oder Umkehr des Druckgebers bei Erreichen des festgelegten und/oder festlegbaren Maximaldrucks beruhen.

Zur Anpassung an konkrete anatomische Gegebenheiten des Trägers der erfindungsgemäßen Halteeinrichtung kann diese in einer bevorzugten Ausgestaltung eine Verstelleinrichtung aufweisen. Mittels der Verstelleinrichtung lässt sich insbesondere die Breite der Halteeinrichtung an die Kopfgröße des Trägers anpassen. Dies ist vor allem bei der Ausgestaltung der Halteeinrichtung als Brillengestell von erheblichem Vorteil in Bezug auf den korrekten Sitz der Halteeinrichtung und die Positionierung des Druckgebers relativ zum Auge. Ferner wird durch eine derartige Einstellmöglichkeit der Tragekomfort für den Träger der Halteeinrichtung erhöht, so dass eine Anwendung auch über längere Zeiträume, beispielsweise zur Langzeiterfassung von ophthalmologischen Daten, beschwerdefrei möglich ist.

Die Verstelleinrichtung kann insbesondere bei einer brillengestellartigen Halteeinrichtung als verstellbarer, insbesondere verschiebbarer und in verschiedenen Positionen fixierbarer, Bügel, vorzugsweise am Rahmen, ausgebildet sein. Im Tragezustand bzw. Verwendungszustand der Halteeinrichtung ist die Verstelleinrichtung vorzugsweise im Bereich der Augenbrauen des Trägers angeordet. Dies erlaubt eine einfache Zugänglichkeit zur Verstelleinrichtung und eine problemlose Einstellung einer optimalen Breite der Halteeinrichtung.

Die Verstelleinrichtung kann je nach Anwendungssituation auf verschiedene Art und Weise ausgebildet sein. Bevorzugte Ausführungsformen umfassen eine Ausgestaltung als Steckhülse, Verstellbügel, Gleitschienensystem und/oder Schraube-Gewinde-System, jeweils ggf. in Verbindung mit Raststufen und/oder einem Fixiermittel, wie beispielsweise einer Schnellverschlussklemme oder einer Fixierschraube.

Im Rahmen der Erfindung kann auch eine mehrteilige Verstelleinrichtung oder mehrere Verstelleinrichtungen, vorzugsweise jeweils den Halteelementen zugeordnet, vorgesehen sein. Im Fall einer Ausgestaltung der Halteeinrichtung als Brillengestell können die Verstelleinrichtungen beispielsweise am Rahmen angeordnet und jeweils einer Seite, d.h. einem Brillenbügel, zugeordnet sein.

Die Verstelleinrichtung kann alternativ oder zusätzlich auch beispielsweise als einzelne, vorzugsweise mittig angeordnete Verstelleinrichtung ausgebildet sein. Hierdurch kann an einer zentralen Stelle auf einfache Weise mit einer einzigen Einstellung eine Anpassung der Form bzw. Größe der Halteeinrichtung vorgenommen werden.

Es ist darüber hinaus eine elektromotorische, pneumatische und/oder hydraulische Anpassung der Halteeinrichtung über die Verstelleinrichtung möglich. Die Verstellung kann in diesem Fall zudem auch mittels einer Fernsteuerung und/oder automatisiert erfolgen.

Wird mittels des Druckgebers ein Druck auf ein Auge mit einer bestimmten Kraft ausgeübt, wirkt dieselbe Kraft auch entgegengerichtet auf den Druckgeber und über diesen auf das Halteelement bzw. die Halteeinrichtung. Aus diesem Grund ist ein ausreichend belastbares Widerlager erforderlich, so dass ein unerwünschtes Verschieben oder Verziehen der Halteeinrichtung verhindert wird und die Druckausübung auf das Auge in wohldefinierter Form erfolgen kann. Bei geringen Drücken sind die auftretenden Kräfte unter Umständen noch so klein, so dass die Halteeinrichtung selbst - abhängig von ihrer Form, Materialart und -stärke - ausreichend starr und festsitzend ist, um der Gegenkraft des Druckgebers entgegenzustehen. Bei fortschreitender Drucksteigerung wird die Halteeinrichtung, insbesondere, wenn diese als Brillengestell ausgebildet ist, jedoch der Gegenkraft nachgeben und sich je nach bestehenden Freiheitsgraden bewegen oder in ihrer Form verändern. Aus diesem Grund wird bei einer bevorzugten Ausführungsform der erfindungsgemäßen Halteeinrichtung ein definiertes Widerlager geschaffen, durch welches die Gegenkraft aufgenommen wird und der Halteeinrichtung ein entsprechender Freiheitsgrad genommen wird, der andernfalls eine unerwünschte Bewegung und/oder Verformung erlauben würde.

Ein solches Widerlager kann in definierter Weise durch ein oder mehrere Anlageelemente dargestellt werden, das bzw. die auf der jeweils kontralateralen Seite gegenüber dem betreffenden Druckgeber angeordnet ist bzw. sind. Ein Anlageelement kann beispielsweise von einem Grundkörper der Halteeinrichtung und/oder eines Halteelements abragend, etwa in Form eines Zapfens oder eines Noppens, ausbebildet sein. Erfindungemäß kann jedoch auch beispielsweise eine Auswölbung, lokale Versteifung oder ein durch die Form der Halteeinrichtung und/oder eines Halteelements ausgezeichneter, insbesondere hervorstehender Punkt als Anlageelement dienen.

Ein Anlageelement kann abnehmbar bzw. austauschbar ausgebildet sein. Auf diese Weise lassen sich verschiedene Anlageelemente an der Halteeinrichtung anbringen, welche letztlich in ihrer Form, Größe und/oder Anzahl an die Anatomie des Trägers der Halteeinrichtung angepasst sind. Ferner kann eine Mehrzahl von Aufnahmemöglichkeiten für ein Anlageelement an verschiedenen Positionen der Halteeinrichtung vorgesehen sein und/oder ein Anlageelement auch an der Halteeinrichtung derart verschiebbar angebracht sein, dass die Position des Anlageelements an die Kopfform des Trägers angepasst werden kann. Darüber hinaus kann auch das Anlageelement selbst größenveränderlich ausgebildet sein, so dass beispielsweise mittels eines Stellgewindes eine Anlage des Anlageelements und damit der Halteeinrichtung als solcher an einem definierten Anlagepunkt am Kopf des Trägers im Tragezustand bewirkt wird. Die Positionsänderung und/oder die Grö-ßenänderung eines Anlageelements kann ferner auch elektromotorisch bewirkt werden und/oder fernsteuerbar und/oder automatisierbar sein.

Idealerweise erfolgt die Abstützung der Halteeinrichtung an einer ausreichend festen Stelle. Diesbezüglich kommt vor allem der Schädelknochen infrage. Es wurde festgestellt, dass bei einer Abstützung der Halteeinrichtung am knöchernen Rand der Augenhöhle (Orbita) auf der dem druckbeaufschlagten Auge gegenüberliegenden Kopfseite eine optimale Kraftaufnahme ermöglicht. Auf diese Weise wird ein geeignetes Widerlager geschaffen, um die Druckausübung auf das betreffende Auge im optimalen Winkel von etwa 90° am Aquator des Augapfels, im temporalen Lidwinkel über das Oberlid und/oder das Unterlid, zu ermöglichen. Auf diese Weise wird auch bei höheren ausgeübten Drücken eine exakte und lagestabile Positionierung der Halteeinrichtung und damit des Druckgebers ermöglicht.

Eine alternative oder zusätzliche Möglichkeit, die Halteeinrichtung gegen die Gegenkraft des Druckgebers abzustützen, kann durch eine symmetrische Druckausübung von zwei gegenüberliegenden Seiten realisiert werden. Die Halteeinrichtung trägt in diesem Fall vorzugsweise wenigstens zwei Druckgeber, die im Tragezustand jeweils auf gegenüberliegenden Kopfseiten angeordnet sind. Bei der Druckbeaufschlagung eines Auges durch einen ersten Druckgeber arbeitet ein gegenüberliegender zweiter Druckgeber synchron in entgegengesetzter Richtung. Die Ausrichtung des zweiten Druckgebers ist vorzugsweise derart, dass sein Kopf, d.h. sein Kontaktelement (ggf. mit einem oder mehreren Kontaktabschnitten) auf den Knochen der Augenhöhlenkante drückt und somit die Kraft des ersten Druckgebers auf das Auge in Bezug auf die Halteeinrichtung ausgleicht, wodurch die gewünschte Lagestabilität erreicht wird.

Die Anordnung von mehreren Druckgebern auf gegenüberliegenden Seiten erlaubt ferner durch geringe Justageeingriffe, welche zudem vorzugsweise automatisierbar sind, eine Umkonfiguration der Dynamometrieanordnung für eine beidseitige Augenuntersuchung.

Die Halteeinrichtung weist in einer bevorzugten Ausführungsform eine Vorrichtung auf, die insbesondere im Tragezustand eine präzise Positionierung des Druckgebers ermöglicht. Hierbei ist vorzugsweise eine Verschiebeeinrichtung zur Bewegung des Druckgebers in distaler Richtung und/oder in vertikaler Richtung vorgesehen. Eine Verschiebung in distaler Richtung bedeutet in diesem Zusammenhang, dass im Tragezustand der Halteeinrichtung eine Bewegung in Blickrichtung, d.h. seitlich entlang des Kopfes zwischen Gesicht und Ohr bzw. Hinterkopf, erfolgt. "Vertikal" bezieht sich dagegen auf die Höhenverstellung der Position des Druckgebers. Besonders vorteilhaft ist das Vorhandensein beider Verstellmöglichkeiten, so dass eine exakte Ausrichtung des Druckgebers relativ zum Auge erfolgen kann.

Die Verschiebeeinrichtung ist vorzugsweise elektromotorisch, pneumatisch und/oder hydraulisch angetrieben und/oder insbesondere fernsteuerbar ausgebildet.

Durch ein Mittel zur Zustandsbestimmung der Verschiebeeinrichtung, beispielsweise einen Positionssensor, kann die absolute Lage des mittels der Verschiebeeinrichtung bewegten Druckgebers erfasst bzw. ausgelesen werden. Dies ermöglicht eine Regelung der Position des Druckgebers im Rahmen eines automatisierten Dynamometrieablaufs. Darüber hinaus können ausgewiesene Punkte, beispielsweise eine personenabhängig optimale Position des Druckgebers relativ zum Auge gespeichert und zu einem späteren Zeitpunkt aufgerufen und insbesondere angefahren werden.

Durch die Möglichkeit, den Druckgeber in zwei Dimensionen relativ zum Kopf einer Person zu verfahren, kann ferner eine Positionierung des Druckgebers am Knochen der Augenhöhlenkante vorgenommen werden. Dieser Druckgeber lässt sich an dieser Stelle als Mittel zur Abstützung der Halteeinrichtung im Sinne eines dynamischen Widerlagers einsetzen, wenn durch einen Druckgeber auf der gegenüberliegenden Kopfseite ein Druck auf das Auge ausgeübt wird.

Der Verfahrweg des Druckkopfes eines Druckgebers bei der Druckausübung auf das Auge ist typischer zweigeteilt und setzt sich zusammen aus dem Verfahrweg aus der Ruhelage bis zum Augenkontakt und einem zusätzlichen Hub von vorzugsweise zwischen 1 mm und 6 mm, bevorzugt zwischen 2 mm und 5 mm.

Der mit dem Druckgeber im Auge hervorgerufene Druck entspricht vorzugsweise einem Gewicht von 1 g bis 20 g, bevorzugt bis 40 g, weiter bevorzugt bis 60 g. In bestimmten Fällen kann jedoch der Druck auch deutlich höher liegen und beispielsweise einem Gewicht von 150 g entsprechen. Die Einwirkzeit liegt vorzugsweise zwischen wenigen Sekunden bis zu 5 min, kann jedoch auch mehr betragen. Die jeweils konkreten Werte sind in hohem Maße abhängig vom jeweiligen Anwendungsfall.

Für verschiedene Anwendungsfälle können die nachfolgenden Daten als Richtwerte dienen, wobei diese in keiner Weise als einschränkend für den Rahmen der Erfindung anzusehen sind.

Bei Venen- und Hirndruckbestimmung: Druckbelastung entsprechend 1 g bis 20 g, Einwirkzeit 10 s bis 2 min; bei arterieller Blutdruckmessung: Druckbelastung entsprechend 40 g bis 140 g, Einwirkzeit 1 min bis 3 min; zur Bestimmung des Abflusswiderstandes mittels OPT zur Glaukomdiagnostik: Druckbelastung entsprechend 30 g bis 50 g, Einwirkzeit 1 min bis 5 min; im Rahmen eines Drucktoleranztests unter Einsatz von optischer Kohärenztomographie: Druckbelastung entsprechend 10 g bis 30 g, Einwirkzeit 2 min bis 4 min; Elektro-Physiologische Untersuchungen: Druckbelastung entsprechend 10 g bis 30 g, Einwirkzeit 1 min bis 5 min.

Die Anfahrtgeschwindigkeit des Dynamometerkopfes liegt vorzugsweise zwischen 0,5 mm/s und 10 mm/s, bevorzugt zwischen 1 mm/s und 5 mm/s.

Die Abschaltung der Druckerhöhung bzw. die Beendigung des Messvorgangs erfolgt bei Erreichen eines vorgegebenen Druckwertes und/oder Ausfahrweges insbesondere automatisch und/oder manuell, beispielsweise auf ein ausgegebenes Signal hin.

Im Fall der Messung des venösen Blutdrucks ist die Besonderheit zu beachten, dass der Gefäßkollaps beim monophasischen Venenkollaps nur einmalig auftritt. und somit nur unzuverlässig detektierbar ist. Eine undulierende Druckausübung führt jedoch zu einem wiederholten Auftreten des Gefäßkollapses. Die Detektierbarkeit des Gefäßkollapses wird somit deutlich erhöht. Zu diesem Zweck kann der Druckgeber zu einer undulierenden Druckausübung ausgebildet sein und/oder eine entsprechend modulierte Ansteuerung des Druckgebers vorgesehen sein. Eine undulierende Druckausübung kann insbesondere auch automatisiert erfolgen. Hierbei kann ein Soll-Mittelwert vorgegeben werden, um den herum der ausgeübte Druck mit einer vorzugsweise einstellbaren Amplitude schwankt. Der Druckbereich, in welchem eine Schwankung erfolgt, liegt vorzugsweise zwischen dem einen Kollaps auslösenden Druckniveau und 3 mmHg, bevorzugt 5 mmHg, niedriger.

Die Halteeinrichtung weist in einer bevorzugten Ausführungsform ein Mittel zur Datenübertragung in Form einer entsprechenden Schnittstelle auf. Die Datenübertragungsschnittstelle ist vorzugsweise zur Übertragung von Steuerdaten und/oder Messdaten, insbesondere einem Druckwert ausgebildet. Es ist somit möglich, den Druckgeber in seiner Funktion und ggf. eine Positioniereinrichtung aus der Distanz anzusteuern und/oder Sensordaten, beispielsweise einen Druckwert, auszulesen, ohne die Halteeinrichtung abnehmen zu müssen. Über die Datenübertragungsschnittstelle kann ferner eine Echtzeit-Steuerung und/oder -Auslesung während der Messung erfolgen. Durch die Datenübertragungsschnittstelle wird also eine Rücckopplungsmöglichkeit der Dynamometrieanordnung zu einer Messanordnung, insbesondere einem OCT-Gerät, geschaffen.

Insbesondere zur Steuerung und/oder zum Auslesen von Messdaten, wie beispielsweise einem auf das Auge ausgeübten Druck, kann mittels einer Datenübertragungsschnittstelle eine Datenübertragung zwischen dem Druckgeber, einem Sensor und/oder der Mess- und Auswertungseinrichtung bzw. einer Messanordnung im Allgemeinen erfolgen. Die übertragenen Daten umfassen dementsprechend vorzugsweise Steuerdaten und/oder Messdaten, insbesondere einen Druckwert. Bei der Messanordnung handelt es sich vorzugsweise um eine optische Kohärenztomographie-Anordnung und/oder eine Scanning Laser Doppler Flowmetrie-Anordnung und/oder eine Laser Speckle Flowmetrie-Anordnung. Durch eine direkte Datenübertragung zwischen einer Einrichtung zur Steuerung und/oder Auswertung und dem Druckgeber bzw. Sensor wird letztlich erst eine Automatisierung des Verfahrens ermöglicht.

Die Schnittstelle ist bevorzugt zur bidirektionalen Datenübertragung ausgebildet.

Die Datenübertragung zwischen dem Druckgeber, dem Sensor und/oder der Mess- und Auswertungseinrichtung bzw. der Messanordnung läuft vorzugsweise drahtlos ab. Hierdurch wird der Aufwand in Bezug auf den Anschluss und die Inbetriebnahmeder Dynamometrieanordnung erheblich reduziert. Eine drahtlose Datenübertragungsschnittstelle ermöglicht ferner eine Kontrolle und Steuerung des Verfahrens, ohne eine zu untersuchende Person in ihrer Bewegungsfreiheit übermäßig einzuschränken. Die Datenübertragungsschnittstelle unterstützt insbesondere verbreitete Protokolle, wie beispielsweise Bluetooth, Wireless LAN, und/oder NFC. Dies gewährleistet neben geringen Kosten für entsprechende Module eine hohe Kompatibilität verschiedener miteinander zusammenwirkender Hardware-Komponenten.

Bevorzugt ist ferner eine Schnittstelle zur Fernsteuerung bzw. -auslesung des Druckgebers, des Sensors und/oder der Mess- und Auswertungseinrichtung, insbesondere über eine Datenfernübertragungsleitung, beispielsweise über das Internet, vorgesehen. Diese erlaubt zum einen medizinischem Fachpersonal die Ausführung bzw. Steuerung des Verfahrens, ohne am Anwendungsort persönlich anwesend zu sein. Zum anderen kann mittels einer Datenfernübertragung eine technische Diagnose und/oder eine Einstellung von Parametern, beispielsweise die Einspielung von Updates, durch technisches Service-Personal aus der Ferne erfolgen.

Vorzugsweise ist die Dynamometrieanordnung derart ausgebildet, dass eine Übertragung und/oder Ausgabe von Daten in Bezug auf die aktuelle Druckausübung, das ablaufende Messprogramm (Venendruckmessung, arterielle Druckmessung, OPT/Glaukomdiagnostik, Drucktoleranzbestimmung am Sehnerv, etc.), eine Rücckopplung der Dynamometrieanordnung zu einer Messanordnung, insbesondere einer OCT-Anordnung und/oder die Zeit zwischen einzelnen Messschritten, beispielsweise bei der OPT zur Glaukomdiagnostik. Ferner kann mittels einer Auswertungseinrichtung eine Auswertung der übermittelten Daten erfolgen, so dass beispielsweise aus dem gemessenen Druckunterschied vor und nach der Belastung bei der OPT direkt ein Wert für die Abflussleichtigkeit ausgegeben wird.

Die Möglichkeiten zur Fernsteuerung und Automatisierung umfassen insbesondere eine Steuerung und/oder ein Auslesen durch einen stationären Computer, einen Tablet-Computer, ein Smartphone oder dergleich und ist vorzugsweise auch über das Internet möglich. Es können so über weite Distanzen Einstellungen vorgenommen und Messungen durchgeführt werden. Über die Datenübertragungsschnittstelle kann medizinisches Fachpersonal und/oder technisches Service-Personal Zugang zur Dynamometrieanordnung und/oder zur Messanordnung erlangen.

Die Möglichkeit zur Automatisierung der Augendynamometrie durch die erfindungsgemäße Halteeinrichtung bzw. Dynamometrieanordnung erlaubt einen Verzicht auf eine andernfalls notwendige Lokalanästhesie des Auges. Durch eine solche Lokalanästhesie wird regelmäßig der Trennfilm auf dem Auge zerstört, was die Sicht in das Auge durch die Pupille für den Arzt und für optische Messgeräte erschwert. Ferner wird durch ein Ausbleiben einer lokalen Betäubung des Auges die Untersuchung für eine Person angenehmer und die Durchführung der Untersuchung zu einer delegierbaren Aufgabe.

Ferner ergibt sich die Möglichkeit einer Nachregulierung bei einem Abfallen des Augendrucks, das im Fall längerer Druckausübung regelmäßig beobachtet wird. Die Erfindung erlaubt somit ein einfaches und vorzugsweise automatisches Konstanthalten des Drucks im Sinne einer sogenannten "Iso-Tonographie". Die Kopplungsmöglichkeit der Dynamometrieanordnung bzw. der Halteeinrichtung mit dem Druckgeber mit einer entsprechenden Messanordnung erlaubt eine Rückkopplung des ausgeübten Drucks mit einer hierdurch induzierten Augeninnendruckveränderung. Überraschenderweise wurde in entsprechenden Versuchen festgestellt, dass zwischen beiden Größen über weite Bereiche ein näherungsweise linearer Zusammenhang besteht.

Der für einen bestimmten Ziel-Augeninnendruck mittels des Druckgebers aufzubringende Druck hängt vor allem von der Anatomie des untersuchten Auges ab. Hierbei spielen insbesondere die Größe des Augapfels und die okuläre Rigidität bzw. die Dicke der Wand des Augapfels eine wesentliche Rolle für dessen Elastizität. Dementsprechend führt beispielsweise nicht stets derselbe ausgeübte Druck zu einem Gefäßkollaps. Eine Kalibration des Systems erfolgt vorzugsweise mittels eines Kontakt-Tonometers über die Hornhaut. Durch die seitliche Druckausübung auf das Auge, die durch die erfindungsgemäße Halteeinrichtung ermöglicht wird, wird die Messung des Augeninnendrucks über die Hornhaut nicht beeinträchtigt. Die vorgenannten individuellen, anatomisch bedingten Unterschiede tragen in niedrigen Druckbereichen weniger stark bei. In höheren Druckbereichen ist jedoch eine Korrektur, beispielsweise mittels eines druckluftbasierten Non-Contact-Tonometers (NCT), in regelmäßigen Zeitintervallen, z.B. alle 30 s oder minütlich, angezeigt.

Die Erfindung ist ferner auf eine Ophthalmodynamometrieanordnung zur Bestimmung des Auftretens eines Gefäßkollapses bei einem Blutgefäß im oder am Auge gerichtet. Mittels der erfindungsgemäßen Ophthalmodynamometrieanordnung lässt sich der Gefäßkollaps insbesondere im Bereich des Eintritts des Gefäßes in den Augapfel und/oder am Austritt aus dem Augapfel ermitteln.

Die Ophthalmodynamometrieanordnung weist in einer einfachsten Ausführungsform einen Druckgeber zum Ausüben eines Drucks auf ein Auge und eine Mess- und Auswertungseinrichtung zur Bestimmung des Auftretens eines Gefäßkollapses bei dem Auge auf.

Erfindungsgemäß ist der Druckgeber der Ophthalmodynamometrieanordnung in der zuvor beschriebenen Weise von einer Halteeinrichtung gehalten bzw. ist mit einer Halteeinrichtung der zuvor beschriebenen Art verbunden. Ein weiterer Aspekt der erfindungsgemäßen Ophthalmodynamometrieanordnung, der zusätzlich zu der Verbindung des Druckgebers mit einer Halteeinrichtung in der zuvor beschriebenen Weise realisiert sein kann, liegt in der Ausbildung der Mess- und Auswertungseinrichtung zur indirekten Bestimmung des Gefäßkollapses aus einer ermittelten Blutflussänderung eines Blutgefäßes am Eintritt in den Augapfel und/oder am Austritt aus dem Augapfel.

Auf die Vorteile eines von einer Halteeinrichtung in der zuvor beschriebenen Weise gehaltenen Druckgebers wurde bereits eingegangen. Es versteht sich, dass bedarfsweise auch die Mess- und Auswertungseinrichtung der Ophthalmodynamometrieanordnung oder Teile von dieser von der Halteeinrichtung gehalten sein können oder mit der Halteeinrichtung verbunden sein können. Dasselbe gilt insbesondere für eine gegebenenfalls separat vom Druckgeber realisierte Druckbestimmungseinrichtung.

Eine Druckbestimmungseinrichtung, mittels derer der auf das Auge ausgeübte Druck messbar, anzeigbar und/oder auf insbesondere indirekte andere Weise ermittelbar ist, kann grundsätzlich auch räumlich getrennt vom Druckgeber und insbesondere nicht mit der Halteeinrichtung verbunden vorgesehen sein.

Bei einer besonders bevorzugten Ausgestaltung der erfindungsgemäßen Ophthalmodynamometrieanordnung ist die Mess- und Auswertungseinrichtung zur Ermittlung der Blutflussänderung mittels eines optischen Verfahrens ausgebildet, wobei der Aufbau vorzugsweise für die Methode der insbesondere zeitlich aufgelösten optischen Kohärenztomographie geeignet ist. Ein Gefäßkollaps an einem Blutgefäß des Auges, insbesondere im Eintritts- bzw. Austrittspunkt der Gefäße nahe dem Eintrittspunkt des Sehnervs in das Auge, d. h. am Papillenrand oder im Papillen-Trichter, lässt sich in diesem Fall sicher bestimmen, selbst bei eingeschränktem visuellen Zugang bzw. bei einem sonst schwer detektierbaren axialen Gefäßkollaps.

Zur Ausführung der Methode der optischen Kohärenztomographie weist die Mess- und Auswertungseinrichtung insbesondere eine Lichtquelle mit vergleichsweise kleiner Kohärenzlänge und mit einem für die Durchdringung des zu untersuchenden Gewebes geeigneten Spektrum auf. Das Licht der Lichtquelle wird für eine OCT-Messung von einem Strahlteiler zum einen durch die Pupille des Auges auf die Netzhaut gelenkt und zum anderen auf einen Reflektor, beispielsweise einen gewöhnlichen Spiegel geleitet.

Das von diesem Reflektor zurückgeworfene und das aus dem Bereich des Augenhintergrunds zurückgestreute Licht passiert erneut den Strahlteiler und gelangt gegebenenfalls über eine entsprechende Abbildungsoptik zu einer Detektionseinrichtung. Die Detektionseinrichtung ist vorzugsweise zur Aufnahme eines zweidimensionalen Bildes ausgebildet, beispielsweise als Kamera oder als optisches Sensorarray. Die einfallenden Strahlen, das heißt der Messstrahl des vom Auge zurückgestreuten Lichts und der vom Reflektor zurückgeworfene Referenzlichtstrahl interferieren nun abhängig von der Struktur des beleuchteten Augenhintergrunds miteinander.

Die Detektionseinrichtung erfasst demnach insbesondere eine zweidimensionale Verteilung von Interferenzinformationen der beiden Strahlen. Hieraus kann letztlich auf die Gewebsstrukturen im betrachteten Augenhintergrund geschlossen werden und es können zudem insbesondere Informationen über die Bewegung des Blutes in den betrachteten Gefäßen, das heißt den Blutfluss, gewonnen werden. Aus einer wiederholten Messung in kurzen Zeitabständen ergibt sich so eine zeitlich aufgelöste Darstellung des betreffenden Gewebes und des Blutflusses in den dortigen Gefäßen. Bei der erfindungsgemäßen Methode ist von Vorteil, dass auf eine quantitative bzw. absolute Blutflussmessung grundsätzlich verzichtet werden kann, da letztlich eine qualitative Detektion einer charakteristischen Veränderung, d.h. des Sistierens, des Blutflusses ausreichend ist, um das Vorliegen eines Gefäßkollapses zu erfassen.

Die Mess- und Auswertungseinrichtung weist zur automatischen Ermittlung eines Sistierens des Blutflusses und damit des Vorliegens eines Gefäßkollapses eine Auswertungseinheit auf, mittels derer die mit der Detektionseinrichtung gewonnenen Informationen manuell, teil- oder vollautomatisch ausgewertet werden können. Die Auswertung der Daten durch die Auswertungseinheit kann insbesondere auch softwareseitig erfolgen. Die notwendigen Mess-Fenster im Rahmen der optischen Detektion werden gegebenenfalls manuell oder automatisch positioniert oder deren automatische Positionierung am Messbild geprüft.

Zur automatischen oder zumindest teilweise automatisierten Durchführung einer ophthalmodynamometrischen Messung bzw. eines ophthalmodynamometrischen Verfahrens und/oder eines Verfahrens der zuvor beschriebenen Art und Weise weist die Ophthalmodynamometrieanordnung vorzugsweise eine Steuereinrichtung auf. Die Steuereinrichtung ist hierzu insbesondere mit dem Druckgeber und der Detektionseinrichtung sowie der Auswertungseinheit gekoppelt. Ferner kann ein Rückkanal von einer Druckbestimmungseinrichtung zur Steuereinrichtung vorgesehen sein, um den ausgeübten Druck auf einen bestimmten Wert einzuregeln. Insbesondere ist vorgesehen, auch bei aufsteigendem dynamometrischem Druck zu messen, was bei pneumatischen Verfahren schwieriger zu realisieren wäre.

Zur automatisierten Durchführung einer Bestimmung des Auftretens eines Gefäßkollapses am Auge kann nun durch die hierzu entsprechend ausgebildete Steuereinrichtung der Druckgeber dazu veranlasst werden, einen steigenden Druck auf das Auge auszuüben, während die Lichtquelle, gegebenenfalls ebenfalls durch die Steuereinrichtung angesteuert, das Auge und den optischen Aufbau der Ophthalmodynamometrieanordnung beleuchtet.

Die Detektionseinrichtung erfasst nun in zeitlich kurzer Folge fortwährend das aus der Struktur des Gewebes im Augenhintergrund resultierende Interferenzbild, das von der Auswertungseinheit auf das Vorliegen eines Sistierens des Blutflusses in den Gefäßen überwacht wird.

Wird durch einen sistierenden Blutfluss ein Gefäßkollaps festgestellt, indem entweder die Auswertungseinheit zu einem entsprechenden Ergebnis gelangt oder indem durch einen Benutzer anhand einer Visualisierung der von der Detektionseinrichtung aufgenommenen Daten der Gefäßkollaps festgestellt wird, kann der von der Druckbestimmungseinrichtung in diesem Moment erfasste Druck auf das Auge ausgelesen, notiert und/oder gespeichert werden. Ferner kann die Auswertungseinheit veranlasst werden, alternativ oder zusätzlich zum ausgeübten Druck auch die Bildinformation bzw. die erfassten Daten der Detektionseinrichtung sowie weitere Parameter, wie beispielsweise Leistung der Lichtquelle und/oder die Zeitinformation der Aufnahme zu speichern.

Die Erfindung wird nachfolgend anhand von Ausführungsbeispielen näher erläutert. Dabei bilden alle beschriebenen und/oder zeichnerisch dargestellten Merkmale für sich oder in beliebiger Kombination den Gegenstand der vorliegenden Erfindung, unabhängig von ihrer Zusammenfassung in den Ansprüchen oder deren Rückbeziehung.

Es zeigt
- Fig. 1: eine perspektivische schematische Darstellung einer bevorzugten Ausführungsform einer erfindungsgemäßen Halteeinrichtung aus Sicht von schräg vorne,
- Fig. 2: eine perspektivische schematische Darstellung der Halteeinrichtung aus Fig. 1 aus Sicht von schräg hinten,
- Fig. 3: eine schematische Darstellung der Halteeinrichtung aus Fig. 1 in Draufsicht,
- Fig. 4: eine schematische Darstellung eines Druckgebers bei der Verwendung,
- Fig. 5: eine schematische Darstellung einer bevorzugten Ausführungsform einer erfindungsgemäßen Ophthalmodynamometrieanordnung,
- Fig. 6: eine schematische Diagrammdarstellung eines typischen zeitlichen Verlaufs des gemessenen Blutflusses in einem Gefäß eines Auges und des auf das Auge ausgeübten Drucks,
- Fig. 7: eine schematische Darstellung, die Druckkompartimente und die Kollapslokalisationen der Zentralnetzhautgefäße zeigt,
- Fig. 8: eine perspektivische schematische Darstellung einer weiteren bevorzugten Ausführungsform einer erfindungsgemäßen Halteeinrichtung aus Sicht von schräg vorne,
- Fig. 9: eine schematische Darstellung einer weiteren bevorzugten Ausführungsform einer erfindungsgemäßen Halteeinrichtung in Draufsicht und
- Fig. 10: eine perspektivische schematische Darstellung einer weiteren bevorzugten Ausführungsform einer erfindungsgemäßen Halteeinrichtung aus Sicht von schräg vorne.

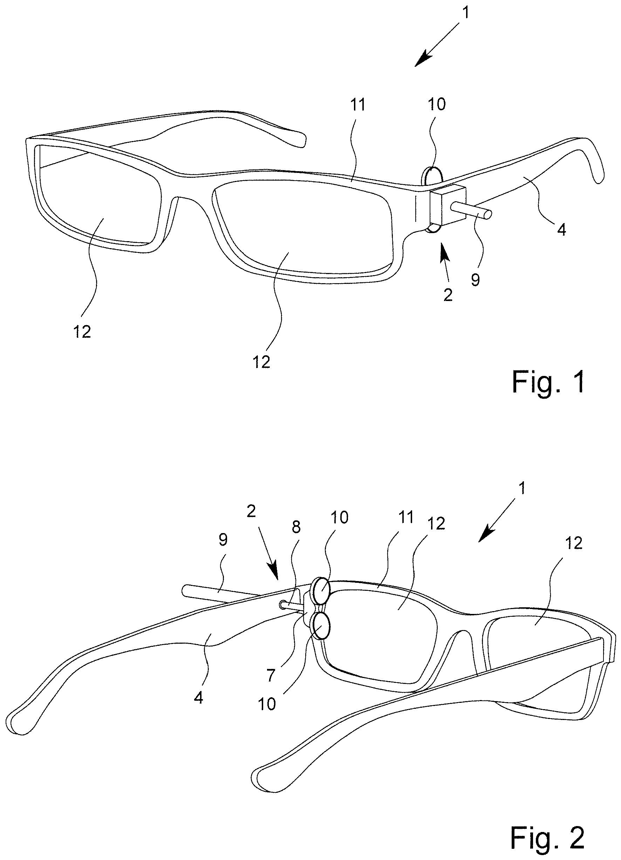

In den Fig. 1 bis 3 ist eine erfindungsgemäße Halteeinrichtung 1 mit einem Druckgeber 2 dargestellt. In der gezeigten Ausführungsform ist die Halteeinrichtung 1 als Brillengestell ausgebildet und lässt sich dadurch leicht an einem nicht dargestellten Kopf einer Person zur Durchführung einer ophthalmodynamometrischen Messung kurzzeitig oder dauerhaft in Position bringen. Hierdurch wird letztlich der Druckgeber 2 in eine optimale Position für eine Druckausübung auf ein in den Fig. 1 bis 3 nicht dargestelltes Auge 3 gebracht.

Der Druckgeber 2 ist hierzu an einem Halteelement 4 angeordnet bzw. mit einem Halteelement 4 verbunden. Das Halteelement 4 ist vorliegend als Brillenbügel ausgebildet, mit dem der Druckgeber 2 fest verbunden ist. Erfindungsgemäß ist auch eine lösbare Verbindung des Druckgebers 2 mit dem Halteelement 4 möglich.

Alternativ oder zusätzlich kann zudem das Halteelement 4 derart ausgestaltet sein, dass es seinerseits zusammen mit dem Druckgeber 2 mit einem Gegenstand, beispielsweise einer vorhandenen Brille, verbindbar ist.

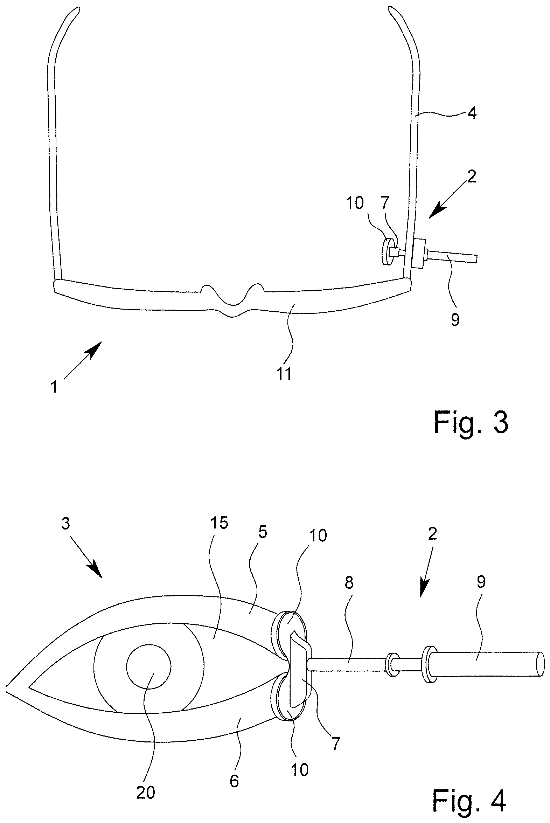

Die Druckausübung auf das Auge 3 durch den Druckgeber 2 erfolgt, wie in Fig. 4 dargestellt, über das Oberlid 5 und/oder das Unterlid 6 des Auges 3 aus seitlicher Richtung. Hierzu weist der Druckgeber 2 ein Kontaktelement 7 auf, das vorliegend gelenkig an einer Kolbenstange 8 gelagert ist, die durch einen Pneumatikzylinder 9 axial bewegbar ist, das heißt in der in Fig. 4 dargestellten Anwendungssituation zum Auge 3 hin sowie in vom Auge 3 wegweisender Richtung.

Das Kontaktelement 7 weist ferner zwei separate Kontaktabschnitte 10 auf, die jeweils zur Anlage an das Oberlid 5 bzw. das Unterlid 6 ausgebildet sind. Die Kontaktelemente 10 können zudem ihrerseits gelenkig gelagert sein.

Alternativ oder zusätzlich zu einer pneumatischen Bewegung des Kontaktelements 7 kann auch eine auf einem rein mechanischen, elektromechanischen und/oder hydraulischen Prinzip beruhende Bewegung vorgesehen sein. Dies ist besonders für die Messung bei ansteigendem Druck von Bedeutung.

Ferner kann die Ausbildung der Kontaktabschnitte 10 von der dargestellten Form als Kontaktplatte abweichen. Abhängig von der konkreten Anwendung kann beispielsweise auch eine Ausbildung als Knauf und/oder als Stößel geeignet sein.

Die Druckausübung über das Oberlid 5 und das Unterlid 6 des Auges 3 erfolgt im Bereich des temporalen Lidwinkels. Eine solche Druckausübung auf das Auge 3 führt bei einer ophthalmodynamometrischen Untersuchung nur zu einer geringen Erhöhung des Drucks im Retrobulbärraum, wobei in anderen Positionen eine Messung des Gefäßdrucks im Retrobulbärraum verfälscht werden würde.

Im einzelnen nicht zeichnerisch dargestellt ist ferner ein Mittel, durch welches der Druckgeber 2 derart ausgebildet ist, dass bei Erreichen eines festgelegten und/oder festlegbaren Maximaldrucks auf das Auge 3 automatisch keine weitere Druckerhöhung durch den Druckgeber 2 erfolgt. Dies dient letztlich dem Schutz des Auges 3 vor einer zu hohen mechanischen Belastung, die im Extremfall zu einer Verletzung oder allgemein zu Schäden im oder am Auge 3 führen könnte.

Im einfachsten Fall kann ein solches Mittel durch einen mechanischen Anschlag realisiert sein, der die axiale Bewegung der Kolbenstange 8 und damit des Kontaktelements 7 auf einen festgelegten Maximalwert begrenzt. Alternativ oder zusätzlich zu einer solchen mechanischen Lösung kann auch der vom Druckgeber 2 ausgeübte Druck durch eine nicht dargestellte Druckbestimmungseinrichtung erfasst werden, die beim Erreichen eines festgelegten und/oder festlegbaren Maximaldrucks den Betrieb des Druckgebers 2 abschaltet und/oder die Bewegung der Kolbenstange 8 und des Kontaktelements 7 umkehrt.

In der dargestellten Ausführungsform der erfindungsgemäßen Halteeinrichtung 1 weist diese weitere, üblicherweise bei einer Brille vorgesehene Merkmale auf, die jedoch für die Verwendung als Halteeinrichtung 1 für einen Druckgeber 2 bei einer ophthalmodynamometrischen Messung nicht von grundsätzlicher Bedeutung sind. Die bekannte Brillenform steigert jedoch die grundsätzliche Akzeptanz bei zu untersuchenden Personen, die Halteeinrichtung 1 anzulegen und zu verwenden.

Ferner lässt sich die Halteeinrichtung 1 auch derart ausbilden, dass neben einem sicheren Sitz der Halteeinrichtung 1 am Kopf einer Person und einer reproduzierbaren Positionierung des Druckgebers 2 relativ zum zu untersuchenden Auge 3 auch beispielsweise Mittel zur Sehkorrektur für die Person integriert werden können. Hierzu weist die Halteeinrichtung 1 in der dargestellten Ausführungsform einen Rahmen 11 auf, der zwei Sichtfenster 12 umgibt, in die beispielsweise Brillengläser zur Sichtkorrektur oder zur Filterung von Licht einer bestimmten Wellenlänge eingesetzt werden können.

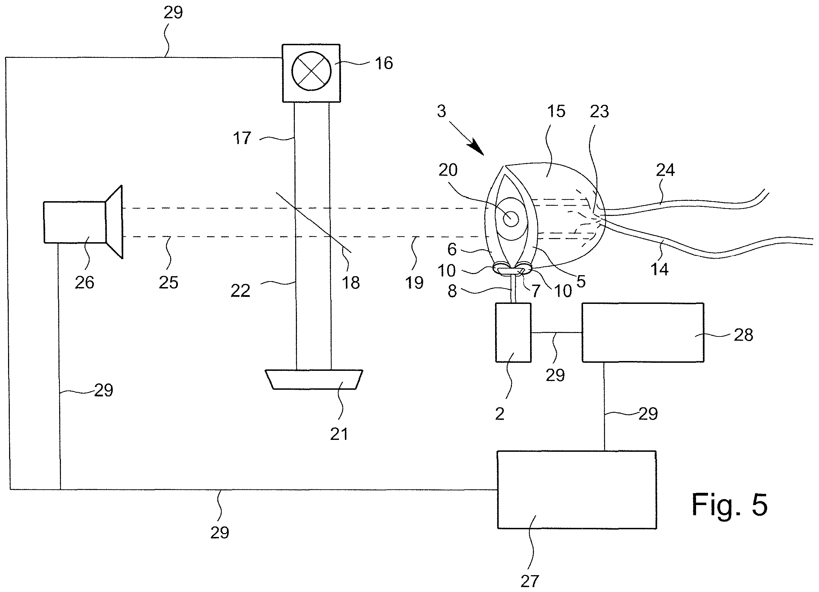

Eine ophthalmodynamometrische Messung am Auge 3 einer Person kann mittels einer erfindungsgemäßen Ophthalmodynamometrieanordnung 13 erfolgen, die in einer bevorzugten Ausführungsform in Fig. 5 dargestellt ist. Die gezeigte Ophthalmodynamometrieanordnung 13 dient letztlich einer sicheren Bestimmung des Auftretens eines Gefäßkollapses bei einem Blutgefäß 14 des Auges 3. Grundsätzlich sind Gefäßkollaps-Analysen auch in anderen Augen-Abschnitten denkbar.

Für eine solche Bestimmung des Auftretens eines Gefäßkollapses wird, vorzugsweise, zunächst vom Druckgeber 2, dessen bevorzugter Aufbau bereits erläutert wurde, mittels des Kontaktelements 7 bzw. der Kontaktabschnitte 10 des Kontaktelements 7 mittelbar Druck auf das Auge 3 über dessen Oberlid 5 und/oder Unterlid 6 ausgeübt. Durch die Druckausübung in seitlicher Richtung auf das Auge 3 im Bereich des temporalen Lidwinkels wird der Augapfel 15 nicht in die nicht dargestellte Augenhöhe gepresst, wodurch der Gewebedruck in der Orbita nicht unnötig erhöht wird und eine dadurch bedingte Fehlmessung vermieden wird.

Zusätzlich zum Druckgeber 2 weist die Ophthalmodynamometrieanordnung 13 eine Mess- und Auswertungseinrichtung auf, die ihrerseits bedarfsweise eine Reihe von Komponenten aufweisen kann.

Die Mess- und Auswertungseinrichtung kann zur indirekten Bestimmung des Gefäßkollapses aus einer ermittelten Blutflussänderung eines Blutgefäßes am Eintritt in den Augapfel 15 und/oder am Austritt aus dem Augapfel 15 ausgebildet sein.

In der in Fig. 5 dargestellten bevorzugten Ausgestaltung der Ophthalmodynamometrieanordnung 13 ist die Mess- und Auswertungseinrichtung zur Anwendung der Methode der optischen Kohärenztomographie (OCT-Angiographie) ausgebildet.

Hierzu weist die Mess- und Auswertungseinrichtung zunächst eine Lichtquelle 16 auf. Die Lichtquelle 16 emittiert vorliegend Licht mit einer spektralen Zusammensetzung, durch die es dem Licht möglich ist, bestrahlte Gewebsschichten im Auge 3 zumindest über eine bestimmte Strecke zu durchdringen. Hierzu kann die Lichtquelle 16 insbesondere Licht mit einem hohen spektralen Anteil im infraroten Bereich emittieren. Das Licht der Lichtquelle 16 sollte ferner zur Durchführung der OCT eine vergleichsweise geringe Kohärenzlänge aufweisen. Dies bedeutet, dass das verwendete Licht relativ breitbandig ist. Zum Beispiel ist eine NIR-Leuchtdiode erheblich breitbandiger als ein NIR-Laser und weist daher eine erheblich kürzere Kohärenzlänge auf. Dies kann nötig sein, da das zu untersuchende Gewebe stark streut. Käme ein NIR-Laser zum Einsatz, könnten die Streuungen im Gewebe zu einer Unzahl von Interferenzen führen, die zu Problemen bei der Auswertung mit dem Detektor führen können.

Das Licht das Lichtquelle 16 wird nun zur Messung als Primärstrahl 17 auf einen Strahlteiler 18, beispielsweise einen halbdurchlässigen Spiegel, geleitet. Der Strahlteiler 18 lenkt nun den Primärstrahl 17 als einfallenden Messstrahl 19 auf das Auge 3, in welches das von der Lichtquelle 16 abgegebene Licht durch die Pupille 20 des Auges 3 eindringt.

Ein Teil des Primärstrahls 17 durchdringt dagegen den Strahlteiler 18 und gelangt zu einem Reflektor 21, beispielsweise einem gewöhnlichen Spiegel, von dem das einfallende Licht als Referenzstrahl 22 unverändert zurückgeworfen wird.

Im Auge 3 wird insbesondere die Papille 23, also der Bereich, in dem der Sehverv 24 in den Augapfel 15 eindringt, beleuchtet. in diesem Bereich dringen auch die Hauptversorgungsgefäße in das Auge 3 ein. Das einfallende Licht wird von den untersuchten Gewebeschichten zurückgestreut. Durch die unterschiedliche Laufzeit bzw. Wegstrecke des Lichts aufgrund der unterschiedlichen Strukturen im bestrahlten Gewebe verlässt das Licht des Messstrahls 19 bereichsweise den Kohärenzbereich relativ zum Referenzstrahl 22. Das als ausfallender Messstrahl 19 zurückgestreute Licht vom Auge 3 gelangt erneut zum Strahlteiler 18, den es durchläuft, wobei es mit dem Referenzstrahl 22, der vom Strahlteiler 18 reflektiert bzw. umgeleitet wird, zu einem resultierenden Strahl 25 vereinigt wird.

Im resultierenden Strahl 25 tritt über seinen Querschnitt bereichsweise Interferenz zwischen dem Licht des Messstrahls 19 und des Referenzstrahls 22 auf. Das Interferenzmuster ist dabei abhängig von den im Auge 3 mit dem Messstrahl 19 bestrahlen Strukturen. Mittels einer nicht dargestellten Abbildungsoptik kann das Licht des Messstrahls 19 bzw. des resultierenden Strahls 25 auf eine Detektionseinrichtung 26 geleitet werden. Hierbei kann die Brennweite der Abbildungsoptik derart gewählt werden, dass eine bestimmte Gewebsschicht im Auge 3 abgebildet wird. Durch ein Durchfahren der Abbildungsebene durch ein Gewebevolumen lässt sich das Gewebevolumen schichtweise als dreidimensionales Tomogramm erfassen. Die Abbildungsoptik, bei der es sich um ein Linsensystem handeln kann, fokussiert das IR-Licht nacheinander auf verschiedene Gewebeschichten. Es entsteht für jede Gewebeschicht ein zweidimensionales Bild. Insgesamt ergibt sich schließlich eine dreidimensionale Volumenabbildung.

Die Phase des als Messstrahl 19 von den Gewebestrukturen im Auge 3 zurückgestreuten Lichts wird ferner auch durch die Bewegung eines abgebildeten Objekts beeinflusst. Aus diesem Grund kann der Blutfluss bzw. die Strömungsbewegung des Blutes in einem Gefäß 14 auf verlässliche Weise erfasst werden. Gelingt es, ein Sistieren des Blutflusses im Gefäß 14 nachzuweisen, so ist dies ein Anzeichen für das Vorliegen eines Gefäßkollapses des betreffenden Gefäßes 14.

Die Detektionseinrichtung 26 ist hierzu vorzugsweise zur Aufnahme eines zweidimensionalen Bildes, insbesondere als Kamera oder als Sensorarray, ausgebildet und ähnelt dem Grunde nach vorzugsweise bekannten Detektionseinrichtungen für OCT-Messungen, die beispielsweise bereits als Fundus-Kamera eingesetzt werden.

Die von der Detektionseinrichtung 26 erfassten Daten bzw. das von der Detektionseinrichtung 26 aufgenommene Bild kann visualisiert werden und/oder digitalisiert und in dieser Form weiterverarbeitet und/oder gespeichert werden.

Zur Verarbeitung der Daten von der Detektionseinrichtung 26 weist die Mess- und Auswertungseinrichtung eine Auswertungseinheit 27 auf. Bei der Auswertungseinheit 27 kann es sich insbesondere um einen Computer, eine elektronische Schaltung oder eine andere Datenverarbeitungseinrichtung handeln. Insbesondere kann die Auswertung der Daten der Detektionseinrichtung 26 durch eine entsprechende Software erfolgen.

Zur Bestimmung eines Gefäßkollapses bei einem Gefäß 14 am Auge 3 wird erfindungsgemäß in kurzen Zeitabständen wiederholt mit der zuvor beschriebenen Apparatur der Augenhintergrund insbesondere im Bereich der Papille 23 erfasst. Den erfassten Daten ist dabei insbesondere der Blutfluss im Gefäß 14 zumindest qualitativ zu entnehmen.

Es wird nun der vom Druckgeber 2 auf das Auge 3 ausgeübte Druck stetig erhöht. Überschreitet infolge der Druckausübung durch den Druckgeber 2 der Umgebungsdruck im Bereich des Gefäßes 14 den Gefäßdruck in dessen Inneren, kommt es zum Kollaps des Gefäßes 14. Während des Gefäßkollapses sistiert der Blutfluss im Inneren des Gefäßes 14. Dies ist in Form einer Änderung des von der Detektionseinrichtung 26 erfassten Bildes bzw. der von der Detektionseinrichtung 26 erfassten Daten feststellbar und kann von der Auswertungseinheit 27 entsprechend ausgewertet und gespeichert werden.

Ferner kann die Auswertungseinheit 27 den beim Auftreten des Gefäßkollapses mittels des Druckgebers 2 auf das Auge 3 ausgeübten Druck speichern. Der vom Druckgeber 2 auf das Auge 3 ausgeübte Druck kann mittels einer nicht im einzelnen dargestellten Druckbestimmungseinrichtung vorgegeben, ausgelesen und/oder indirekt ermittelt werden.

Durch eine entsprechende Kalibration kann aus dem vom Druckgeber 2 auf das Auge 3 ausgeübten Druck im Moment des Auftretens des Gefäßkollapses der Gefäßdruck ermittelt werden. Insbesondere durch eine Messung des venösen Gefäßdrucks kann ferner auch verlässlich auf den Hirndruck geschlossen werden. Insbesondere die dafür notwendige Bestimmung eines venösen Gefäßkollapses ist mittels bekannter visueller Methoden nur auf unzuverlässige Weise möglich oder in einigen Fällen sogar unmöglich.

Zur automatischen oder zumindest teilweise automatisierten Durchführung des zuvor beschriebenen ophthalmodynamometrischen Verfahrens kann die Ophthalmodynamometrieanordnung 13, wie in der in Fig. 5 dargestellten Form, eine Steuereinrichtung 28 aufweisen. Die Steuereinrichtung 28 dient in diesem Fall insbesondere der Steuerung des Druckgebers 2, indem der vom Druckgeber 2 auf das Auge 3 ausgeübte Druck von der Steuereinrichtung 28 vorgegeben bzw. eingestellt wird.

Vorzugsweise ist die Steuereinrichtung 28 über einen Rückkanal mit einer Druckbestimmungseinrichtung verbunden, mittels derer der tatsächliche momentan auf das Auge 3 ausgeübte Druck erfasst werden kann. Dadurch ist eine gezielte Regelung des Druckgebers 2 in Bezug auf den auf das Auge 3 ausgeübten Druck durch die Steuereinrichtung 28 möglich.

Die Steuereinrichtung 28 kann ferner die Detektionseinrichtung 26 in Bezug auf die Datenerfassung, insbesondere hinsichtlich der für die zeitliche Auflösung der Messung relevante Frequenz der Datenerfassung, ansteuern. Darüber hinaus ist auch eine Steuerung der Lichtquelle 16 sowie der Auswertungseinheit 27 erfindungsgemäß möglich.

Das Zusammenwirken der einzelnen Komponenten der Mess- und Auswertungseinrichtung wird insbesondere durch eine Verbindung der Komponenten über Daten- bzw. Steuerleitungen 29 ermöglicht. Es versteht sich, dass die Daten-/Steuerleitungen 29 nicht als leitende Verbindung ausgebildet sein müssen, sondern beispielsweise auch durch eine drahtlose Übertragung von Daten und/oder Steuersignalen realisiert sein können.

Der Druckgeber 2 ist bei der erfindungsgemäßen Ophthalmodynamometrieanordnung 13 mit einer erfindungsgemäßen Halteeinrichtung 1, vorzugsweise in einer grundsätzlichen Ausgestaltung gemäß der Darstellung der Fig. 1 bis 3, verbunden und wird von dieser in optimaler Position relativ zum zu untersuchenden Auge 3 gehalten.

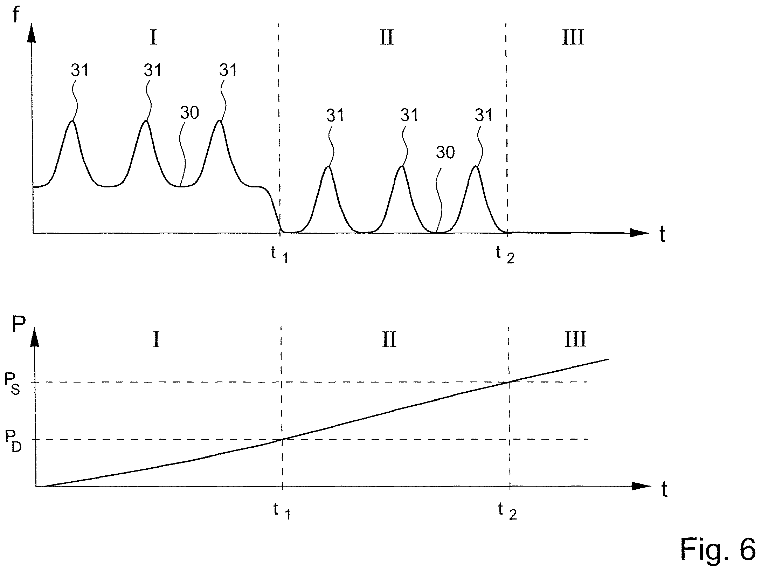

In Fig. 6 ist schematisch ein beispielhafter zeitlicher Verlauf der bei einer Messung zur Bestimmung des Auftretens eines Gefäßkollapses ermittelten Daten gezeigt. Das obere Diagramm in Fig. 6 entspricht dabei dem aus den Daten der Detektionseinrichtung 26 ermittelten Blutfluss f im zeitlichen Verlauf. Eine absolute Quantifizierung des Blutflusses f ist hierbei nicht zwingend erforderlich

Das untere Diagramm in Fig. 6 zeigt in identischer Zeitskala wie das obere Diagramm den Verlauf des infolge einer steigenden Druckausübung durch den Druckgeber 2 auf das Auge 3 in der Umgebung des Gefäßes 14 vorliegenden Drucks P, der durch Kalibration aus dem von außen auf das Auge 3 ausgeübten Druck bestimmt werden kann.

Es ist erkennbar, dass ausgehend von einem Basisdruckwert der Druck P am Gefäß 14 infolge der steigenden Druckausübung durch den Druckgeber 2 auf das Auge 3 stetig ansteigt. Der Blutfluss f im Gefäß 14 alterniert zunächst zwischen einem Plateau 30, das einen gewissen Grundfluss des Blutes im Gefäß 14 widerspiegelt, und über das Plateau 30 hinausgehenden Pulsen 31. Die Pulse 31 zeigen einen beschleunigten Blutfluss f im Gefäß 14 infolge der Pumpaktivität des Herzens an.

Steigt nun der Umgebungsdruck P am Gefäß 14 am Zeitpunkt t₁ auf einen Wert an, der dem diastolischen Gefäßdruck P_{D} entspricht, sinkt der Blutfluss f im Bereich des Plateaus 30 zwischen den Pulsen 31 im wesentlichen auf null ab. Der Blutfluss f sistiert also zwischen den Pulsen 31. Der Gefäßdruck ist demnach nicht mehr ausreichend groß, um dem Umgebungsdruck P im Bereich des Gefäßes 14 entgegenzustehen, so dass das Gefäß 14 kollabiert. Durch die Pumpaktivität des Herzens wird jedoch der Gefäßdruck kurzzeitig pulsweise derart erhöht, dass der Umgebungsdruck P kurzzeitig überschritten wird, so dass der Gefäßkollaps kurzzeitig aufgehoben wird und das Blut im Gefäß 14 in dieser Zeit weiterfließt.

Wird der Umgebungsdruck infolge einer steigenden Druckausübung durch den Druckgeber 2 auf das Auge 3 weiter erhöht, so entspricht der Umgebungsdruck P am Gefäß 14 zum Zeitpunkt t₂ dem systolischen Gefäßdruck Ps. Nach Überschreiten dieses Drucks reicht der Gefäßdruck auch während der Pumpaktivität des Herzens nicht mehr aus, um das kollabierte Gefäß aufzuweiten. Der Blutfluss f kommt demnach ab dem Zeitpunkt t₂ vollständig zum Erliegen, wie aus dem oberen Diagramm in Fig. 6 hervorgeht.

Die Zeitpunkte t₁, t₂ des Erreichens der charakteristischen Gefäßdrücke P_{D}, Ps unterteilen den zeitlichen Verlauf der ermittelten Daten somit in drei Abschnitte I, II, III. Im ersten Abschnitt I beträgt der Umgebungsdruck P am untersuchten Gefäß 14 weniger als der diastolische Gefäßdruck P_{D}. Dementsprechend ist ein ungehinderter Blutfluss möglich. Im zweiten Abschnitt II liegt der Umgebungsdruck P am Gefäß 14 zwischen dem diastolischen Gefäßdruck P_{D} und dem systolischen Gefäßdruck Ps. Ein Blutfluss f ist demnach nur kurzzeitig während der Pumpaktivität des Herzens in Form von Pulsen 31 zu messen. Im dritten Abschnitt III ist schließlich der Umgebungsdruck P am Gefäß 14 größer als der systolische Gefäßdruck Ps, der während einer Pumpaktivität des Herzens vorliegt. Demnach wird ein vollständiges Sistieren des Blutflusses erfasst.

Die Übergänge zwischen den Abschnitten I, II, III sind anhand des zeitlichen Verlaufs des Blutflusses f deutlich erkennbar und legen charakteristische Zeitpunkte t₁ und t₂ fest. Eine entsprechende Kalibrierung vorausgesetzt sind schließlich aus dem zu den Zeitpunkten t₁ und t₂ jeweils vorliegenden Umgebungsdruck P am Gefäß 14 charakteristische Gefäßdruckwerte in Form des diastolischen Gefäßdrucks P_{D} und des systolischen Gefäßdrucks Ps ermittelbar.

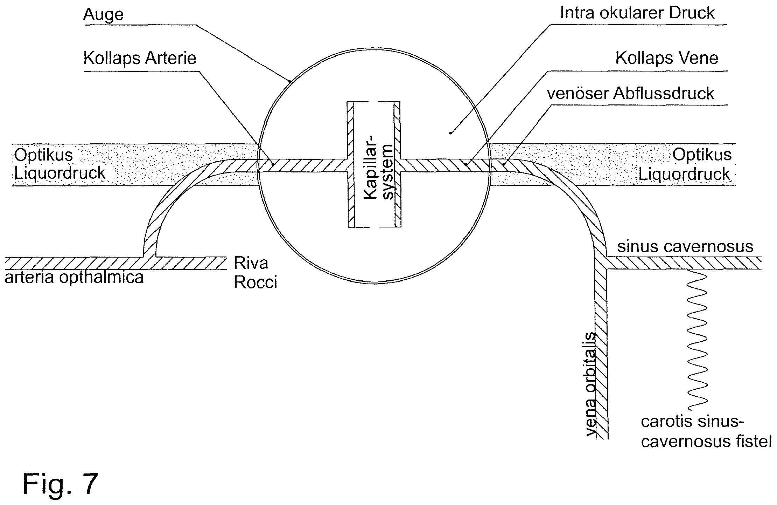

In Fig. 7 sind schematisch die Druckkompartimente und die Kollapslokalisationen der zentralen Netzhautgefäße dargestellt.

Im Gegensatz zur Zentral-Arterie (zentrale Netzhautarterie) handelt es sich im Venensystem (auch Zentralvene bzw. zentrale Netzhautvene) um ein Niederdrucksystem. In den meisten Fällen ist ein ähnliches Kollapsverhalten der Zentralvene wie bei der Zentral-Arterie zu erwarten. Hier gelten die analogen Bedingungen für den Gefäßkollaps wie bei der Zentral-Arterie.

Abweichend vom arteriellen Kollaps sind hier Sonderformen des Gefäßkollapses nach den klinischen Beobachtungen zu erwarten. Dennoch wird sich auch hier ein Gefäßkollaps mit Hilfe der OCT Flussanalyse nachweisen lassen.

Da der Venendruck maßgeblich vom Hirndruck verantwortet wird, ist mit Hilfe des Venendruckes auch eine Hirndruckmessung möglich. Die Feststellung eines spontanen Venenkollapses mit Hilfe der Kollaps-Gefäß-Analyse reicht zur Feststellung eines nicht-erhöhten Hirndruckes bereits aus, auch ohne dynamometrisches Verfahren. Insbesondere lediglich in den Fällen, in denen kein spontaner Venenkollaps vorliegt, kann die Dynamometrie wie oben beschrieben durchgeführt werden.

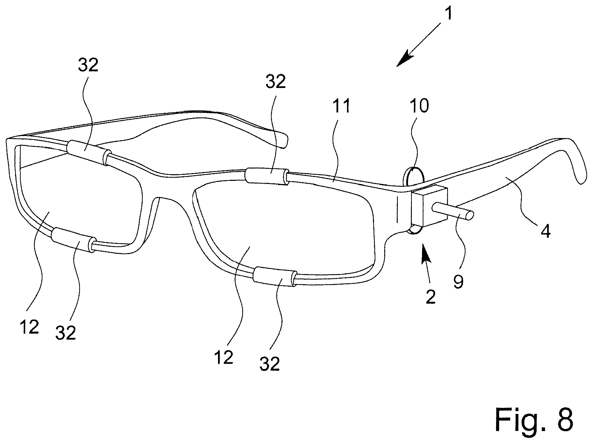

In Fig. 8 ist eine weitere bevorzugte Ausgestaltung der erfindungsgemäßen Halteeinrichtung 1 gezeigt. In dort dargestellten Beispiel entspricht die Halteeinrichtung 1 vom grundsätzlichen Aufbau her dem Ausführungsbeispiel gemäß Fig. 1. Vorliegend weißt die Halteeinrichtung 1 allerdings ferner eine Verstelleinrichtung 32 auf, mittels derer ihre Größe, vorliegend in Bezug auf die Breite, verändert werden kann.

In der vorliegenden Darstellung sind mehrere Verstelleinrichtungen 32 im Rahmen 11 der Halteeinrichtung 1 vorgesehen. Die Anordnung ist jedoch letztlich vom Anwendungsfall abhängig und kann grundsätzlich an beliebiger Stelle, beispielsweise mittig, realisiert sein.

Im dargestellten Beispiel ist die Verstelleinrichtung 32 jeweils als hülsenförmiges Element ausgebildet, in welchem Teile des Rahmens 11 derart insbesondere teleskopartig verschieblich angeordnet sind, dass die brillengestellartige Halteeinrichtung 1 zur Anpassung an eine bestimmte Kopfform in ihrer Breite durch Ziehen und Drücken verändert werden kann, wobei verschiedene Einstellungen durch nicht im Detail dargestellte Raststufen im Innern der Verstelleinrichtung 32 fixiert werden. Alternativ oder zusätzlich kann auch eine Ausgestaltung der Verstelleinrichtung 32 beispielsweise als Bügel, Schienensystem und/oder Stellschraube vorgesehen sein.

Durch die Anordnung der Verstelleinrichtungen 32 im Bereich des Rahmens 11 sind diese auf einfache Weise zugänglich und ermöglichen so eine problemlose Justage der Halteeinrichtung 1 an die Kopfform des Trägers.

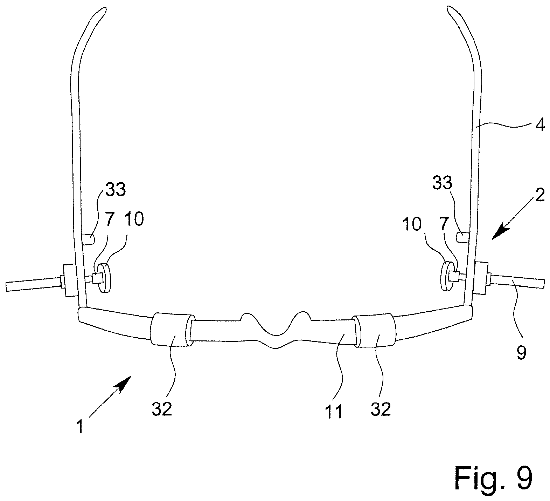

Fig. 9 zeigt ein weiteres bevorzugtes Ausführungsbeispiel der erfindungsgemäßen Halteeinrichtung 1, die in der dortigen Darstellung ebenfalls als Brillengestell ausgebildet ist. Abweichend zur in Fig. 3 gezeigten Ausführungsform trägt die Halteeinrichtung 1 vorliegend jedoch Druckgeber 2 auf beiden Seiten, d.h. an beiden als Brillenbügel ausgebildeten Halteelementen 4. Ein solcher symmetrischer Aufbau ermöglicht eine Untersuchung beider Augen 3 mit geringem Justageaufwand, insbesondere ohne einen aufwendigen Wechsel des Druckgebers 2 von einer auf die andere Kopfseite bzw. ohne das Erfordernis, eine zweite Halteeinrichtung 1 zur Untersuchung des zweiten Auges 3 bereitzuhalten.

Ferner kann bei einer Druckausübung auf das Auge 3 durch einen der Druckgeber 2 bei einer entsprechenden Positionierung des jeweils anderen Druckgebers 2 am Knochen, insbesondere am Rand der Augenhöhle, ein entsprechender Gegendruck in gleichem Maß aufgebaut werden, so dass der ausgeübte Druck nur in der gewünschten Weise auf das Auge 3 wirkt, ohne die Halteeinrichtung 1 entgegen der Druckausübungsrichtung des Druckgebers 2, d.h. insbesondere entgegen der Bewegung der Kolbenstange 8, zu verschieben.

Im Ausführungsbeispiel der Fig. 9 sind darüber hinaus Anlageelemente 33 zur Anlage am Schädelknochen, vorzugsweise am Orbitarand, dargestellt. Die Anlageelemente 33 dienen vorliegend als statisches Widerlager, um die Gegenkraft bei Ausübung eines Drucks auf das Auge 3 mittels des gegenüber dem Anlageelement 33 angeordneten Druckgebers 2 aufzunehmen.

Ein oder mehrere Anlageelemente 33 können unabhängig von einer symmetrischen Ausgestaltung der Halteeinrichtung 1 mit beidseitigen Druckgebern 2, aber auch in Kombination mit dieser vorgesehen sein und sich gegenseitig ergänzen, wie im Beispiel der Fig. 9 gezeigt.

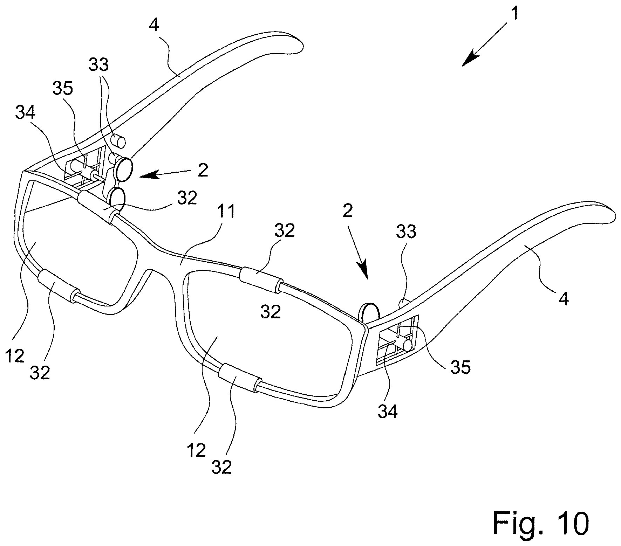

Fig. 10 zeigt ein weiteres Ausführungsbeispiel der erfindungsgemäßen Halteeinrichtung 1. Auch bei diesem Beispiel sind zwei Druckgeber 2 beidseitig vorgesehen. Hiervon unabhängig liegt die Besonderheit bei dieser bevorzugten Ausführungsform darin, dass der Druckgeber 2 jeweils verschieblich gelagert ist und von einer ersten Verschiebeeinrichtung 34 in distaler Richtung verschoben werden kann. Davon unabhängig kann er zudem von einer zweiten Verschiebeeinrichtung 35 in vertikaler Richtung verschoben werden. Dies erlaubt eine exakte Positionierung des Druckgebers 2 relativ zum entsprechenden Auge 3, so dass die Kontaktierung des Auges 3 mit dem Kontaktelement 7 bzw. mit den Kontaktabschnitten 10 an der optimalen Stelle, vorzugsweise am temporalen Lidwinkel über das Oberlid 5 und/oder das Unterlid 6, erfolgt.

Mittels der Verschiebeeinrichtugen 34, 35 lässt sich der Druckgeber 2 zudem an eine Stelle verfahren, an welcher er in optimaler Weise eine Gegenkraft zum ausgeübten Druck des gegenüberliegenden Druckgebers 2 im Sinne eines dynamischen Widerlagers ausüben kann. Eine geeignete Stelle hierzu liegt, wie zuvor beschrieben, am Rand der Augenhöhle im Bereich des Augapfeläquators.

Es kann ferner eine Einstellung des Winkels des Druckgebers 2 zum Auge 3 vorgenommen werden. Eine entsprechende Vorrichtung, beispielsweise ein Gelenk, ist jedoch aus Gründen der Übersichtlichkeit der Zeichnung nicht im Einzelnen darstellt.

Die vorgestellten Ausführungsbeispiele dienen lediglich zur beispielhaften Veranschaulichung der Merkmale bevorzugter Ausgestaltungen der erfindungsgemäßen Halteeinrichtung 1, insbesondere in der Funktion als Dynamometrieanordnung.

Dargestellte Merkmale können in beliebiger Kombination und unabhängig voneinander realisiert sein.

Insbesondere kann andere Ausgestaltung der Halteeinrichtung 1 als die in Form Brillengestells vorgesehen sein oder andere Art von Brillengestell als das dargestellte eingesetzt werden.

Nicht im Einzelnen dargestellt ist ferner eine Datenübertragungseinrichtung in Form einer Datenübertragungsschnittstelle. Insbesondere bei einer bevorzugten Ausgestaltung als drahtlose Datenübertragungsschnittstelle kann diese auch in die Halteeinrichtung 1, in den vorliegenden Beispielen vorzugsweise in den Rahmen 11 oder in ein Halteelement 4, integriert werden.

### Bezugszeichenliste:

| | | | |
|---|---|---|---|
| 1 | Halteeinrichtung | 24 | Sehnerv |
| 2 | Druckgeber | 25 | resultierender Strahl |
| 3 | Auge | 26 | Detektionseinrichtung |
| 4 | Halteelement | 27 | Auswertungseinheit |
| 5 | Oberlid | 28 | Steuereinrichtung |
| 6 | Unterlid | 29 | Daten-/Steuerleitung |
| 7 | Kontaktelement | 30 | Plateau |
| 8 | Kolbenstange | 31 | Puls |
| 9 | Pneumatikzylinder | 32 | Verstelleinrichtung |
| 10 | Kontaktabschnitt | 33 | Anlageelement |
| 11 | Rahmen | 34 | Verschiebeeinrichtung (distal) |
| 12 | Sichtfenster | 35 | Verschiebeeinrichtung (vertikal) |
| 13 | Ophthalmodynamometrieanordnung | t | Zeit |
| 14 | Blutgefäß | t₁ | Zeitpunkt |
| 15 | Augapfel | t₂ | Zeitpunkt |
| 16 | Lichtquelle | f | Blutfluss |
| 17 | Primärstrahl | P | Druck |
| 18 | Strahlteiler | P_{D} | diastolischer Druck |
| 19 | Messstrahl | Ps | systolischer Druck |
| 20 | Pupille | | |
| 21 | Reflektor | I | erster Abschnitt |
| 22 | Referenzstrahl | II | zweiter Abschnitt |
| 23 | Papille | III | dritter Abschnitt |

## Patentansprüche

1. Halteeinrichtung (1), insbesondere zur Verwendung bei der Ophthalmodynamometrie, mit einem Druckgeber (2) und einem Halteelement (4), wobei der Druckgeber (2) ein mechanisch, hydraulisch und/oder pneumatisch bewegbares, als Knauf, Stößel und/oder Kontaktplatte ausgebildetes Kontaktelement (7) zur Anlage gegen das Oberlid (5) und/oder das Unterlid (6) im temporalen Lidwinkel aufweist und am Halteelement (4) derart gehalten ist, dass in einem Verwendungszustand der Halteeinrichtung (1) mittels des Druckgebers (2) Druck auf ein Auge (3) aus seitlicher Richtung über ein Oberlid (5) und/oder ein Unterlid (6) im temporalen Lidwinkels des Auges (3) zumindest im Wesentlichen quer zur Blickachse des Auges (3) ausgeübt werden kann.

2. Halteeinrichtung nach Anspruch 1, **dadurch gekennzeichnet, dass** die Halteeinrichtung (1) im Verwendungszustand portabel, insbesondere tragbar, vorzugsweise in der Art einer Brille, ausgebildet ist und/oder dass das Halteelement (4) als Brillenbügel oder zur Verbindung mit einem Brillenbügel ausgebildet ist.

3. Halteeinrichtung nach Anspruch 1 oder 2, **dadurch gekennzeichnet, dass** das Kontaktelement (7) gelenkig gelagert ist und/oder eine Mehrzahl von getrennten Kontaktabschnitten (10), vorzugsweise zwei zur Anlage gegen das Auge (3) oder gegen Teile des Auges (3), vorzugsweise das Oberlid (5) und/oder das Unterlid (6) des Auges (3), bestimmte Kontaktabschnitte (10), aufweist, und/oder dass der Druckgeber (2) derart ausgebildet ist, dass bei Erreichen eines festgelegten und/oder festlegbaren Maximaldrucks auf das Auge (3) automatisch keine weitere Druckerhöhung durch den Druckgeber (2) erfolgt.

4. Halteeinrichtung nach einem der vorhergehenden Ansprüche, **dadurch gekennzeichnet, dass** eine Verstelleinrichtung (32) zur Änderung der Form und/oder Größe, vorzugsweise der Breite, der Halteeinrichtung (1), insbesondere eines Rahmens (11) der Halteeinrichtung (1), vorgesehen ist.

5. Halteeinrichtung nach einem der vorhergehenden Ansprüche, **dadurch gekennzeichnet, dass** das Halteelement (4) wenigstens eine Verschiebeeinrichtung (34, 35) zur vertikalen und/oder distalen Verschiebung des Druckgebers (2) aufweist.

6. Halteeinrichtung nach einem der vorhergehenden Ansprüche, **dadurch gekennzeichnet, dass** wenigstens ein Anlageelement (33) zur Anlage an einem Anlagepunkt, insbesondere an einem Schädelknochen, vorzugsweise an einem Orbitarand, vorgesehen ist.

7. Halteeinrichtung nach einem der Ansprüche 3 bis 6, **dadurch gekennzeichnet, dass** wenigstens ein Kontaktabschnitt (10) eine vorzugsweise konvexe Krümmung aufweist.

8. Halteeinrichtung nach einem der vorhergehenden Ansprüche, **dadurch gekennzeichnet, dass** eine vorzugsweise drahtlose Datenübertragungsschnittstelle vorgesehen ist, wobei die Datenübertragungsschnittstelle vorzugsweise zur bidirektionalen Datenübertragung und/oder zur Übertragung von Steuerdaten und/oder Messwerten, vorzugsweise Daten eines Drucksensors, ausgebildet ist, und wobei, vorzugsweise, die Datenübertragungsschnittstelle zur Kopplung der Halteeinrichtung (1) mit einer Messanordnung, insbesondere einer optischen Kohärenztomographie-Anordnung, einer Scanning Laser Doppler Flowmetrie-Anordnung und/oder einer Laser Speckle Flowmetrie-Anordnung, ausgebildet ist.

9. Halteeinrichtung nach einem der vorhergehenden Ansprüche, **dadurch gekennzeichnet, dass** ein Fixierband vorgesehen ist und/oder die Halteeinrichtung zumindest teilweise helmartig ausgebildet ist.

10. Halteeinrichtung nach einem der vorhergehenden Ansprüche, **dadurch gekennzeichnet, dass** eine insbesondere einen Drucksensor aufweisende Druckbestimmungseinrichtung vorgesehen ist, wobei die Druckbestimmungseinrichtung, vorzugsweise, vom Druckgeber (2) in Bezug auf die Druckerzeugung entkoppelt ist.

11. Halteeinrichtung nach einem der vorhergehenden Ansprüche, **dadurch gekennzeichnet, dass** der Druckgeber (2) zu einer undulierenden Druckausübung ausgebildet ist.

12. Verfahren zur Bestimmung des Abflusswiderstandes für Kammerwasser eines Auges (3), wobei der Innendruck des Auges (3) bestimmt wird, eine Druckbeaufschlagung des Auges (3) mittels einer Dynamometrieanordnung erfolgt, nach der Druckbeaufschlagung der Innendruck des Auges (3) erneut bestimmt wird und aus dem Unterschied der gemessenen Innendrücke ein Wert für den Abflusswiderstand bestimmt wird,
**dadurch gekennzeichnet,**
**dass** die Dynamometrieanordnung als Halteeinrichtung (1) nach einem der Ansprüche 1 bis 11 ausgebildet ist oder eine Halteeinrichtung (1) nach einem der Ansprüche 1 bis 11 aufweist.

13. Verwendung einer Halteeinrichtung nach einem der Ansprüche 1 bis 11 zur Okulopressions-Tonometrie und/oder zur Blutdruckbestimmung, insbesondere in Verbindung mit optischer Kohärenztomographie, und/oder zur Bestimmung der papillären Mikrooszillation, insbesondere in Verbindung mit optischer Kohärenztomographie, und/oder zur elektro-physiologischen Untersuchung, jeweils an einem Auge (3).

14. Ophthalmodynamometrieanordnung (13) zur Bestimmung des Auftretens eines Gefäßkollapses bei einem Blutgefäß (14) im oder am Auge (3), insbesondere am Eintritt in den Augapfel (15) und/oder am Austritt aus dem Augapfel (15), mit einem Druckgeber (2) zum Ausüben eines Drucks auf ein Auge (3) und einer Mess- und Auswertungseinrichtung zur Bestimmung des Auftretens eines Gefäßkollapses bei dem Auge (3),
**dadurch gekennzeichnet,**
**dass** der Druckgeber (2) mit einer Halteeinrichtung (1) nach einem der Ansprüche 1 bis 11 verbunden ist.

15. Ophthalmodynamometrieanordnung nach Anspruch 14, **dadurch gekennzeichnet, dass** die Mess- und Auswertungseinrichtung zur Ermittlung der Blutflussänderung mittels eines optischen Verfahrens, vorzugsweise mittels insbesondere zeitlich aufgelöster optischer Kohärenztomographie (OCT), ausgebildet ist.

## Claims

1. Holding device (1), in particular for use in ophthalmodynamometry , having a pressure transmitter (2) and a holding element (4), wherein the pressure transmitter (2) comprises a mechanically, hydraulically and/or pneumatically movable contact element (7) in the form of a knob, plunger and/or contact plate for installation against the upper eyelid (5) and/or the lower eyelid (6) in the temporal eyelid angle and is held on the holding element (4) in such a way that, in a state of use of the holding device (1), pressure can be exerted by means of the pressure transmitter (2) on an eye (3) from the lateral direction via an upper eyelid (5) and/or a lower eyelid (6) in the temporal eyelid angle of the eye (3) at least crosswise to the visual axis of the eye (3).

2. Holding device according to claim 1, **characterized in that** the holding device (1) in the state of use is designed to be portable, in particular wearable, preferably in the manner of eyeglasses, and/or **in that** the holding element (4) is designed as a spectacle temple or for connection to a spectacle temple.

3. Holding device according to claim 1 or 2, **characterized in that** the contact element (7) is articulated and/or has a plurality of separate contact sections (10), preferably two for installation against the eye (3) or against parts of the eye (3), preferably the upper eyelid (5) and/or the lower eyelid (6) of the eye (3), contact sections (10), and/or that the pressure transmitter (2) is designed in such a way that when a fixed and/or determinable maximum pressure on the eye (3) is reached, no further increase in pressure is automatically effected by the pressure transmitter (2).

4. Holding device according to one of the preceding claims, **characterized in that** an adjusting device (32) is provided for changing the shape and/or size, preferably the width, of the holding device (1), in particular of a frame (11) of the holding device (1).

5. Holding device according to one of the preceding claims , **characterized in that** the holding element (4) comprises at least one displacement device (34, 35) for vertically and/or distally displacing the pressure transmitter (2).

6. Holding device according to one of the preceding claims , **characterized in that** at least one abutment element (33) is provided for abutting on an abutting point, in particular on a skull bone, preferably on an orbital rim.

7. Holding device according to one of the claims 3 to 6, **characterized in that** at least one contact section (10) comprises a preferred convex curvature.

8. Holding device according to one of the preceding claims, **characterized in that** a preferably wireless data transmission interface is provided, wherein the data transmission interface is preferably designed for bidirectional data transmission and/or for the transmission of control data and/or measurement values, preferably data from a pressure sensor, and wherein, preferably, the data transmission interface is designed for coupling the holding device (1) to a measuring arrangement, in particular wherein it is designed for coupling to an optical coherence tomography arrangement, a scanning laser Doppler flowmetry arrangement and/or a laser speckle flowmetry arrangement.

9. Holding device according to one of the preceding claims, **characterized in that** a fixing strap is provided and/or the holding device is at least partially designed in the shape of a helmet.

10. Holding device according to one of the preceding claims , **characterized in that** a pressure determination device comprising, in particular, a pressure sensor is provided, wherein the pressure determination device is, preferably, decoupled from the pressure transmitter (2) with respect to the pressure generation.

11. Holding device according to one of the preceding claims, **characterized in that** the pressure transmitter (2) is designed to exert undulating pressure.

12. Method for determining the outflow resistance for aqueous humor of an eye (3), wherein the internal pressure of the eye (3) is determined, the eye (3) is pressurized by means of a dynamometry arrangement, the internal pressure of the eye (3) is determined again after the pressurization and a value for the outflow resistance is determined from the difference between the measured internal pressures,
**characterised in that**
the dynamometry arrangement is designed as a holding device (1) according to one of the claims 1 to 11 or comprises a holding device (1) according to one of the claims 1 to 11.

13. Use of a holding device according to one of the claims 1 to 11 for oculopres-sion tonometry and/or for determining blood pressure, in particular in connection with optical coherence tomography, and/or for determining papillary microoscillation, in particular in connection with optical coherence tomography, and/or for electrophysiological examination, respectively at an eye (3).

14. Ophthalmodynamometry arrangement (13) for determining the occurrence of a vascular collapse in a blood vessel (14) in or on the eye (3), in particular at the entry into the eyeball (15) and/or at the exit from the eyeball (15), having a pressure transmitter (2) for exerting a pressure on an eye (3) and a measuring and evaluation device for determining the occurrence of a vascular collapse in the eye (3),
**characterised in that**
the pressure transmitter (2) is connected to a holding device (1) according to one of the claims 1 to 11.

15. Ophthalmodynamometry arrangement according to claim 14, **characterized in that** the measuring and evaluation device is designed to determine the blood flow change by means of an optical method, preferably by means of, in particular, time-resolved optical coherence tomography (OCT).

## Revendications

1. Dispositif de maintien (1), en particulier pour l'utilisation en ophtalmodynamométrie, avec un transmetteur de pression (2) et un élément de maintien (4), le transmetteur de pression (2) présentant un élément de contact (7) mobile mécaniquement, hydrauliquement et/ou pneumatiquement, réalisé sous forme de pommeau, de coulisseau et/ou de plaque de contact, pour l'appui contre la paupière supérieure (5) et/ou la paupière inférieure (6) dans l'angle palpébral temporal et étant maintenu sur l'élément de maintien (4) de telle manière, que, dans un état d'utilisation du dispositif de maintien (1), une pression peut être exercée au moyen du transmetteur de pression (2) sur un oeil (3) à partir d'une direction latérale par l'intermédiaire d'une paupière supérieure (5) et/ou d'une paupière inférieure (6) dans l'angle palpébral temporal de l'œil (3) au moins essentiellement transversalement à l'axe de vision de l'œil (3).

2. Dispositif de maintien selon la revendication 1, **caractérisé en ce que** le dispositif de maintien (1) est conçu, à l'état d'utilisation, de manière portable, en particulier de manière à pouvoir être porté, de préférence à la manière d'une paire de lunettes, et/ou **en ce que** l'élément de maintien (4) est conçu comme une branche de lunettes ou pour être relié à une branche de lunettes.

3. Dispositif de maintien selon la revendication 1 ou 2, **caractérisé en ce que** l'élément de contact (7) est monté de manière articulée et/ou comporte une pluralité de sections de contact séparées (10), de préférence deux sections de contact (10) pour s'appliquer contre l'œil (3) ou contre des parties de l'œil (3), de préférence la paupière supérieure (5) et/ou la paupière inférieure (6) de l'œil (3), et/ou **en ce que** le transmetteur de pression (2) est conçu de telle sorte que, lorsqu'une pression maximale déterminée et/ou pouvant être déterminée est atteinte sur l'œil (3), aucune autre augmentation de pression n'est automatiquement effectuée par le transmetteur de pression (2).

4. Dispositif de maintien selon l'une des revendications précédentes, **caractérisé en ce qu'**il est prévu un dispositif de réglage (32) pour modifier la forme et/ou la taille, de préférence la largeur, du dispositif de maintien (1), en particulier d'un cadre (11) du dispositif de maintien (1).

5. Dispositif de retenue selon l'une des revendications précédentes, **caractérisé en ce que** l'élément de maintien (4) présente au moins un dispositif de déplacement (34, 35) pour le déplacement vertical et/ou distal du transmetteur de pression (2).

6. Dispositif de maintien selon l'une des revendications précédentes, **caractérisé en ce qu'**au moins un élément d'appui (33) est prévu pour s'appuyer sur un point d'appui, notamment sur un os du crâne, de préférence sur un rebord orbitaire.

7. Dispositif de maintien selon l'une des revendications 3 à 6, **caractérisé en ce qu'**au moins une portion de contact (10) présente une courbure de préférence convexe.

8. Dispositif de maintien selon l'une des revendications précédentes, **caractérisé en ce qu'**une interface de transmission de données de préférence sans fil est prévue, l'interface de transmission de données étant de préférence conçue pour la transmission bidirectionnelle de données et/ou pour la transmission de données de commande et/ou de valeurs de mesure, de préférence de données d'un transmetteur de pression, et dans lequel, de préférence, l'interface de transmission de données est conçue pour coupler le dispositif de maintien (1) à un dispositif de mesure, en particulier un dispositif de tomographie par cohérence optique, un dispositif de débitmétrie Doppler à balayage laser et/ou un dispositif de débitmétrie speckle à laser.

9. Dispositif de maintien selon l'une des revendications précédentes, **caractérisé en ce qu'**il est prévu une bande de fixation et/ou **en ce que** le dispositif de maintien est réalisé au moins partiellement en forme de casque.

10. Dispositif de maintien selon l'une des revendications précédentes, **caractérisé en ce qu'**il est prévu un dispositif de détermination de la pression présentant en particulier un capteur de pression, le dispositif de détermination de la pression étant, de préférence, découplé du transmetteur de pression (2) par rapport à la génération de pression.

11. Dispositif de retenue selon l'une des revendications précédentes, **caractérisé en ce que** le transmetteur de pression (2) est conçu pour exercer une pression ondulant.

12. Procédé de détermination de la résistance à l'écoulement de l'humeur aqueuse d'un oeil (3), dans lequel la pression interne de l'œil (3) est déterminée, une mise sous pression de l'œil (3) est effectuée au moyen d'un dispositif dynamométrique, la pression interne de l'œil (3) est à nouveau déterminée après la mise sous pression et une valeur de la résistance à l'écoulement est déterminée à partir de la différence des pressions internes mesurées,
**caractérisé en ce que**
l'agencement dynamométrique est conçu comme un dispositif de maintien (1) selon l'une des revendications 1 à 11 ou présente un dispositif de maintien (1) selon l'une des revendications 1 à 11.

13. Utilisation d'un dispositif de maintien selon l'une des revendications 1 à 11 pour la tonométrie d'oculopression et/ou pour la détermination de la pression artérielle, notamment en liaison avec la tomographie par cohérence optique, et/ou pour la détermination de la microoscillation papillaire, notamment en liaison avec la tomographie par cohérence optique, et/ou pour l'examen électrophysiologique, respectivement sur un oeil (3).

14. Dispositif d'ophtalmodynamométrie (13) pour déterminer l'apparition d'un collapsus vasculaire au niveau d'un vaisseau sanguin (14) dans ou sur l'œil (3), en particulier à l'entrée dans le globe oculaire (15) et/ou à la sortie du globe oculaire (15), comprenant un transmetteur de pression (2) pour exercer une pression sur un oeil (3) et un dispositif de mesure et d'évaluation pour déterminer l'apparition d'un collapsus vasculaire au niveau de l'œil (3),
**caractérisé en ce que**
le transmetteur de pression (2) est relié à un dispositif de maintien (1) selon l'une des revendications 1 à 11.

15. Dispositif d'ophtalmodynamométrie selon la revendication 14, **caractérisé en ce que** le dispositif de mesure et d'évaluation est conçu pour déterminer la variation du flux sanguin au moyen d'un procédé optique, de préférence au moyen d'une tomographie par cohérence optique (OCT) résolue en particulier dans le temps.
